# EUROPÄISCHE PATENTANMELDUNG

(11) **EP 0 722 943 A1**
(43) Veröffentlichungstag der Anmeldung: **24.07.1996**
(21) Anmeldenummer: 96100153.4
(22) Anmeldetag: 08.01.1996
(51) Int. Cl.: C07D 473/00, C07D 473/30, A61K 31/52

(54) **9-substituierte 2-(2-n-Alkoxyphenyl)-purin-6-one**

(30) Priorität: 19.01.1995 DE 19501480
(71) Anmelder: BAYER AG, 51368 Leverkusen (DE)
(72) Erfinder: Niewöhner, Ulrich, Dr., D-42929 Wermelskirchen (DE); Bischoff, Erwin, Dr., D-42115 Wuppertal (DE); Schütz, Helmuth, Dr., D-42115 Wuppertal (DE); Perzborn, Elisabeth, Dr., D-42327 Wuppertal (DE); Schramm, Matthias, Dr., D-51375 Leverkusen (DE)

(57) **Zusammenfassung**

9-Substituierte 2-(2-n-Alkoxyphenyl)purin-6-one werden hergestellt indem man entsprechend substituierte Imidazole cyclisiert und die so erhaltenen Purine derivatisiert. Die 9-substituierten 2-(2-n-Alkoxyphenyl)prin-6-one können als Wirkstoffe in Arzneimitteln eingesetzt werden, insbesondere in Arzneimitteln zur Behandlung von Entzündungen, thromboembolischen Erkrankungen sowie Herz-/Kreislauferkrankungen.

## Beschreibung

Die vorliegende Erfindung betrifft 9-substituierte 2-(2-n-Alkoxyphenyl)-purin-6-one, Verfahren zur Herstellung und ihre Verwendung in Arzneimitteln, insbesondere zur Behandlung von Entzündungen, thromboembolischen, sowie Herz- und Kreislauferkrankungen und Erkrankungen des Urogenitalsystems.

Aus den Publikationen WO 94 /00453 und WO 93/12095 sind Purinone und Quinazolinone mit einer selektiven cGMP PDE inhibitorischen Wirkung bekannt.

Phosphodiesterasen (PDE's) spielen eine wesentliche Rolle in der Regulation des intrazellulären cGMP und cAMP-Spiegels. Von den bisher beschriebenen Phosphodiesterase-Isoenzymgruppen PDE I bis PDE V [Nomenklatur nach Beavo and Reifsnyder (vgl. Beavo, J.A. and Reifsnyder, D.H., Trends in Pharmacol. Sci. 11, 150-155 (1990))] sind die Ca-Calmodulin aktivierte PDE I, die cGMP stimulierbare PDE II und die cGMP spezifische PDE V im wesentlichen für den Metabolismus von cGMP verantwortlich. Aufgrund der unterschliedlichen Verteilung dieser cGMP metabolisierenden PDE's im Gewebe sollten selektive Inhibitoren je nach Gewebsverteilung des entsprechenden Isoenzyms die cGMP-Spiegel im entsprechenden Gewebe anheben. Dieses kann zu einer spezifischen, antiaggregatorischen, antispastischen, gefäßdilatierenden, antiarrhythmischen und/oder antiinflammatorischen Wirkung führen.

Die vorliegende Erfindung betrifft nun 9-substituierte 2-(2-n-Alkoxyphenyl)-purin-6-one der allgemeinen Formel (I), 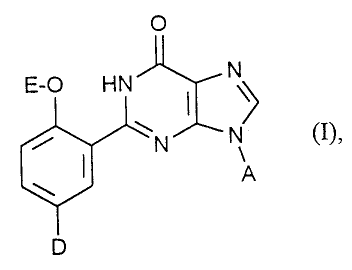
in welcher
- A: für einen Rest der Formel 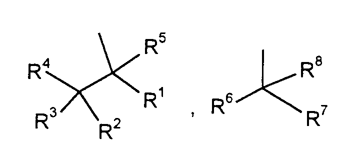 oder -(CH₂)ₐ-CH₃
steht,
worin
- a: eine Zahl 9, 10, 11, 12, 13, 14 oder 15 bedeutet,
- R¹: geradkettiges oder verzweigtes Alkyl mit 2 bis 10 Kohlenstoffatomen bedeutet, das gegebenenfalls durch Phenyl substituiert ist, das seinerseits durch Halogen, Nitro, Cyano, durch geradkettiges oder verzweigtes Alkyl mit bis zu 6 Kohlenstoffatomen oder durch eine Gruppe der Formel -SO₂-NR⁹R¹⁰ substituiert sein kann,
worin
- R⁹ und R¹⁰: gleich oder verschieden sind und
Wasserstoff Phenyl oder geradkettiges oder verzweigtes Alkyl mit bis zu 6 Kohlenstoffatomen bedeuten, das gegebenenfalls durch Hydroxy substituiert ist,
oder gemeinsam mit dem Stickstoffatom einen 5- bis 6-gliedrigen, gesättigten Heterocyclus mit bis zu 2 weiteren Heteroatomen aus der Reihe S, N und/oder O bilden, der gegebenenfalls, auch über eine freie N-Funktion, durch geradkettiges oder verzweigtes Alkyl mit bis zu 6 Kohlenstoffatomen substituiert ist, das seinerseits durch Hydroxy substituiert sein kann,
und/oder
Alkyl gegebenenfalls durch eine Gruppe der Formel -NR¹¹R¹² substituiert ist,
worin
- R¹¹ und R¹²: die oben angegebene Bedeutung von R⁹ und R¹⁰ haben und mit dieser gleich oder verschieden sind,
- R²: Wasserstoff, Azido, geradkettiges oder verzweigtes Alkyl mit bis zu 6 Kohlenstoffatomen oder eine Gruppe der Formel -OR¹³, O-SO₂R¹⁴ oder -NR¹⁵R¹⁶ bedeutet,
worin
- R¹³: Wasserstoff, eine Hydroxyschutzgruppe, geradkettiges oder verzweigtes Acyl, mit bis zu 6 Kohlenstoffatomen, Benzoyl oder geradkettiges oder verzweigtes Alkyl mit bis zu 6 Kohlenstoffatomen bedeutet, das gegebenenfalls durch Carboxy oder geradkettiges oder verzweigtes Alkoxycarbonyl mit bis zu 6 Kohlenstoffatomen oder durch eine Gruppe der Formel -CO-NR¹⁷R¹⁸ substituiert ist,
worin
- R¹⁷ und R¹⁸: gleich oder verschieden sind und Wasserstoff oder geradkettiges oder verzweigtes Alkyl mit bis zu 4 Kohlenstoffatomen bedeuten,
- R¹⁴: geradkettiges oder verzweigtes Alkyl mit bis zu 4 Kohlenstoffatomen oder Phenyl bedeutet,
- R¹⁵ und R¹⁶: gleich oder verschieden sind und Wasserstoff, eine Aminoschutzgruppe, geradkettiges oder verzweigtes Alkyl oder Acyl mit jeweils bis zu 6 Kohlenstoffatomen, Formyl, Benzoyl oder eine Gruppe der Formel -SO₂R¹⁹ bedeuten,
worin
- R¹⁹: die oben angegebene Bedeutung von R¹⁴ hat und mit dieser gleich oder verschieden ist,
- R³: Wasserstoff bedeutet,
oder
- R² und R³: gemeinsam einen Rest der Formel =O oder -=N-OR²⁰ bilden,
worin
- R²⁰: Wasserstoff oder geradkettiges oder verzweigtes Alkyl mit bis zu 6 Kohlenstoffatomen bedeutet, das gegebenenfalls durch Phenyl oder durch eine Gruppe der Formel -NR²¹R²² substituiert ist,
worin
- R²¹ und R²²: gleich oder verschieden sind und Wasserstoff, Phenyl oder geradkettiges oder verzweigtes Alkyl mit bis zu 6 Kohlenstoffatomen bedeuten,
- R⁴: Wasserstoff oder geradkettiges oder verzweigtes Alkyl mit bis zu 4 Kohlenstoffatomen bedeutet,
- R⁵ und R⁸: gleich oder verschieden sind und Wasserstoff oder geradkettiges oder verzweigtes Alkyl mit bis zu 3 Kohlenstoffatomen bedeuten,
- R⁶: Wasserstoff oder geradkettiges oder verzweigtes Alkyl mit bis zu 5 Kohlenstoffatomen bedeutet, das gegebenenfalls durch Hydroxy substituiert ist,
- R⁷: geradkettiges oder verzweigtes Alkyl mit 2 bis 8 Kohlenstoffatomen bedeutet, das durch eine Gruppe der Formel -NR²³R²⁴ substituiert ist,
worin
- R²³ und R²⁴: gleich oder verschieden sind und Wasserstoff oder geradkettiges oder verzweigtes Alkyl mit bis zu 5 Kohlenstoffatomen bedeuten, das gegebenenfalls durch Hydroxy substituiert ist,
oder durch Phenyl substituiert ist, das seinerseits durch die Gruppe der Formel -SO₂-NR²⁵R²⁶ substituiert ist,
worin
- R²⁵ und R²⁶: die oben angegebene Bedeutung von R⁹ und R¹⁰ haben,
- D: für Wasserstoff oder für eine Gruppe der Formel -SO₂-NR²⁷R²⁸ steht,
worin
- R²⁷ und R²⁸: gleich oder verschieden sind und die oben angegebene Bedeutung von R⁹ und R¹⁰ haben und mit dieser gleich oder verschieden sind,
- E: für geradkettiges oder verzweigtes Alkyl mit bis zu 8 Kohlenstoffatomen steht
und deren Tautomere und Salze.

Die erfindungsgemäßen Stoffe können auch als Salze vorliegen. Im Rahmen der Erfindung sind physiologisch unbedenkliche Salze bevorzugt.

Physiologisch unbedenkliche Salze können Salze der erfindungsgemäßen Verbindungen mit anorganischen oder organischen Säuren sein. Bevorzugt werden Salze mit anorganischen Säuren wie beispielsweise Salzsäure, Bromwasserstoffsäure, Phosphorsäure oder Schwefelsäure, oder Salze mit organischen Carbon- oder Sulfonsäuren wie beispielsweise Essigsäure, Maleinsäure, Fumarsäure, Äpfelsäure, Zitronensäure, Weinsäure, Milchsäure, Benzoesäure, oder Methansulfonsäure, Ethansulfonsäure, Phenylsulfonsäure, Toluolsulfonsäure oder Naphthalindisulfonsäure.

Die erfindungsgemäßen Verbindungen der allgemeinen Formel (I) können in verschiedenen stereochemischen Formen auftreten, die sich entweder wie Bild und Spiegelbild (Enantiomere), oder die sich nicht wie Bild und Spiegelbild (Diastereomere) verhalten. Die Erfindung betrifft sowohl die Antipoden als auch die Racemformen sowie die Diastereomerengemische. Die Racemformen lassen sich ebenso wie die Diastereomeren in bekannter Weise in die stereoisomer einheitlichen Bestandteile trennen.

Ein über das Stickstoffatom gebundener, 5- bis 6-gliedriger gesättigter Heterocyclus, der außerdem als Heteroatome bis zu 2 Sauerstoff-, Schwefel- und/oder Stickstoffatome enthalten kann, steht im allgemeinen für Piperidyl, Morpholinyl, Piperazinyl oder Pyrrolidinyl. Besonders bevorzugt sind Piperidyl und Morpholinyl.

Hydroxyschutzgruppe im Rahmen der oben angegebenen Definition steht im allgemeinen für eine Schutzgruppe aus der Reihe: Trimethylsilyl, Triethylsilyl, Triisopropylsilyl, tert.Butyl-dimethylsilyl, Triphenylsilyl oder Benzyl. Bevorzugt sind Trimethylsilyl, tert.Butyl-dimethylsilyl oder Benzyl.

Aminoschutzgruppe im Rahmen der Erfindung sind die üblichen in der Peptid-Chemie verwendeten Aminoschutzgruppen.

Hierzu gehören bevorzugt: Benzyloxycarbonyl, Methoxycarbonyl, Ethoxycarbonyl, Propoxycarbonyl, Isopropoxycarbonyl, Butoxycarbonyl, Isobutoxycarbonyl, tert.-Butoxycarbonyl, Formyl oder Acetyl.

Bevorzugt sind Verbindungen der allgemeinen Formel (I),
in welcher
- A: für einen Rest der Formel 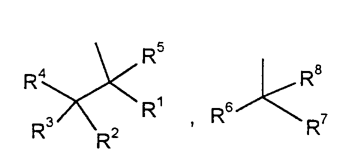 oder -(CH₂)ₐ-CH₃
steht,
worin
- a: eine Zahl 9, 10, 11, 12 oder 13 bedeutet,
- R¹: geradkettiges oder verzweigtes Alkyl mit 2 bis 8 Kohlenstoffatomen bedeutet, das gegebenenfalls durch Phenyl substituiert ist, das seinerseits durch Fluor, Chlor, Brom, Nitro, Cyano, durch geradkettiges oder verzweigtes Alkyl mit bis zu 4 Kohlenstoffatomen oder durch eine Gruppe der Formel -SO₂-NR⁹R¹⁰ substituiert sein kann,
worin
- R⁹ und R¹⁰: gleich oder verschieden sind und Wasserstoff, Phenyl oder geradkettiges oder verzweigtes Alkyl mit bis zu 5 Kohlenstoffatomen bedeuten, das gegebenenfalls durch Hydroxy substituiert ist,
oder gemeinsam mit dem Stickstoffatom einen Morpholinyl, Pyrrolidinyl-, Piperidinylring oder einen Piperazinylring bilden, der gegebenenfalls, auch über eine freie NH-Funktion, durch geradkettiges oder verzweigtes Alkyl mit bis zu 3 Kohlenstoffatomen substituiert ist, das seinerseits durch Hydroxy substituiert sein kann,
und/oder
Alkyl gegebenenfalls durch eine Gruppe der Formel -NR¹¹R¹² substituiert ist,
worin
- R¹¹ und R¹²: die oben angegebene Bedeutung von R⁹ und R¹⁰ haben und mit dieser gleich oder verschieden sind,
- R²: Wasserstoff, Azido, geradkettiges oder verzweigtes Alkyl mit bis zu 4 Kohlenstoffatomen oder eine Gruppe der Formel -OR¹³, -O-SO₂-R¹⁴ oder -NR¹⁵R¹⁶ bedeutet,
worin
- R¹³: Wasserstoff, Benzyl, geradkettiges oder verzweigtes Acyl mit bis zu 4 Kohlenstoffatomen, Benzoyl oder geradkettiges oder verzweigtes Alkyl mit bis zu 4 Kohlenstoffatomen bedeutet, das gegebenenfalls durch Carboxy oder geradkettiges oder verzweigtes Alkoxycarbonyl mit bis zu 4 Kohlenstoffatomen oder durch eine Gruppe der Formel -CO-NR¹⁷R¹⁸ substituiert ist,
worin
- R¹⁷ und R¹⁸: gleich oder verschieden sind und Wasserstoff, oder geradkettiges oder verzweigtes Alkyl mit bis zu 3 Kohlenstoffatomen bedeuten,
- R¹⁴: geradkettiges oder verzweigtes Alkyl mit bis zu 3 Kohlenstoffatomen oder Phenyl bedeutet,
- R¹⁵ und R¹⁶: gleich oder verschieden sind und Wasserstoff, tert. Butoxycarbonyl, Benzyloxycarbonyl oder geradkettiges oder verzweigtes Alkyl oder Acyl mit jeweils bis zu 4 Kohlenstoffatomen, Formyl, Benzoyl oder eine Gruppe der Formel -SO₂R¹⁹ bedeuten,
worin
- R¹⁹: die oben angegebene Bedeutung von R¹⁴ hat und mit dieser gleich oder verschieden ist,
- R³: Wasserstoff bedeutet,
oder
- R² und R³: gemeinsam einen Rest der Formel =O oder -=N-OR²⁰ bilden,
worin
- R²⁰: Wasserstoff oder geradkettiges oder verzweigtes Alkyl mit bis zu 4 Kohlenstoffatomen bedeutet, das gegebenenfalls durch Phenyl oder durch eine Gruppe der Formel -NR²¹R²² substituiert ist,
worin
- R²¹ und R²²: gleich oder verschieden sind und Wasserstoff, Phenyl oder geradkettiges oder verzweigtes Alkyl mit bis zu 4 Kohlenstoffatomen bedeuten,
- R⁴: Wasserstoff oder geradkettiges oder verzweigtes Alkyl mit bis zu 3 Kohlenstoffatomen bedeutet,
- R⁵ und R⁸: gleich oder verschieden sind und Wasserstoff oder Methyl bedeuten,
- R⁶: Wasserstoff oder geradkettiges oder verzweigtes Alkyl mit bis zu 3 Kohlenstoffatomen bedeutet, das gegebenenfalls durch Hydroxy substituiert ist,
- R⁷: geradkettiges oder verzweigtes Alkyl mit 2 bis 6 Kohlenstoffatomen bedeutet, das durch eine Gruppe der Formel -NR²³R²⁴ substituiert ist,
worin
- R²³ und R²⁴: gleich oder verschieden sind und Wasserstoff oder geradkettiges oder verzweigtes Alkyl mit bis zu 4 Kohlenstoffatomen bedeuten, das gegebenenfalls durch Hydroxy substituiert ist,
oder durch Phenyl substituiert ist, das seinerseits durch eine Gruppe der Formel -SO₂-NR²⁵R²⁶ substituiert ist,
worin
- R²⁵ und R²⁶: gleich oder verschieden sind und die oben angegebene Bedeutung von R⁹ und R¹⁰ haben,
- D: für Wasserstoff oder für eine Gruppe der Formel -SO₂-NR²⁷R²⁸ steht,
worin
- R²⁷ und R²⁸: gleich oder verschieden sind und die oben angegebene Bedeutung von R⁹ und R¹⁰ haben und mit dieser gleich oder verschieden sind,
- E: für geradkettiges oder verzweigtes Alkyl mit bis zu 6 Kohlenstoffatomen steht,
und deren Tautomere und Salze.

Besonders bevorzugt sind Verbindungen der allgemeinen Formel (I),
in welcher
- A: für einen Rest der Formel 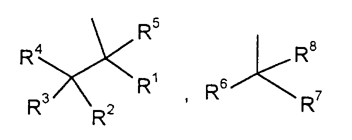 oder -(CH₂)ₐ-CH₃
steht,
worin
- a: eine Zahl 9, 10, 11 oder 12 bedeutet,
- R¹: geradkettiges oder verzweigtes Alkyl mit 2 bis 7 Kohlenstoffatomen bedeutet, das gegebenenfalls durch Phenyl substituiert ist, das seinerseits durch Fluor, Chlor, Brom, Nitro, Cyano, durch geradkettiges oder verzweigtes Alkyl mit bis zu 3 Kohlenstoffatomen oder durch eine Gruppe der Formel -SO₂-NR⁹R¹⁰ substituiert sein kann,
worin
- R⁹ und R¹⁰: gleich oder verschieden sind und Wasserstoff, Phenyl oder geradkettiges oder verzweigtes Alkyl mit bis zu 4 Kohlenstoffatomen bedeuten, das gegebenenfalls durch Hydroxy substituiert ist,
oder gemeinsam mit dem Stickstoffatom einen Morpholinyl, Pyrrolidinyl-, Piperidinylring oder einen Piperazinylring bilden, der gegebenenfalls, auch über eine freie NH-Funktion, durch geradkettiges oder verzweigtes Alkyl mit bis zu 3 Kohlenstoffatomen substituiert ist, das seinerseits durch Hydroxy substituiert sein kann,
und/oder
Alkyl gegebenenfalls durch eine Gruppe der Formel -NR¹¹R¹² substituiert ist,
worin
- R¹¹ und R¹²: die oben angegebene Bedeutung von R⁹ und R¹⁰ haben und mit dieser gleich oder verschieden sind,
- R²: Wasserstoff, Azido, geradkettiges oder verzweigtes Alkyl mit bis zu 3 Kohlenstoffatomen oder eine Gruppe der Formel -OR¹³, -OSO₂R¹⁴ oder -NR¹⁵R¹⁶ bedeutet,
worin
- R¹³: Wasserstoff, geradkettiges oder verzweigtes Acyl mit bis zu 3 Kohlenstoffatomen, Benzoyl oder geradkettiges oder verzweigtes Alkyl mit bis zu 3 Kohlenstoffatomen bedeutet, das gegebenenfalls durch Carboxy oder geradkettiges oder verzweigtes Alkoxycarbonyl mit bis zu 3 Kohlenstoffatomen oder durch eine Gruppe der Formel -CO-NR¹⁷R¹⁸ substituiert ist,
worin
- R¹⁷ und R¹⁸: gleich oder verschieden sind und Wasserstoff, Methyl oder Ethyl bedeuten,
- R¹⁴: geradkettiges oder verzweigtes Alkyl mit bis zu 3 Kohlenstoffatomen oder Phenyl bedeutet,
- R¹⁵ und R¹⁶: gleich oder verschieden sind und Wasserstoff, tert. Butoxycarbonyl, geradkettiges oder verzweigtes Alkyl oder Acyl mit jeweils bis zu 3 Kohlenstoffatomen, Formyl, Benzoyl oder eine Gruppe der Formel -SO₂R¹⁹ bedeuten,
worin
- R¹⁹: die oben angegebene Bedeutung von R¹⁴ hat und mit dieser gleich oder verschieden ist,
- R³: Wasserstoff bedeutet,
oder
- R² und R³: gemeinsam einen Rest der Formel =O oder -=N-OR²⁰ bilden,
worin
- R²⁰: Wasserstoff oder geradkettiges oder verzweigtes Alkyl mit bis zu 4 Kohlenstoffatomen bedeutet, das gegebenenfalls durch Phenyl oder durch eine Gruppe der Formel -NR²¹R²² substituiert ist,
worin
- R²¹ und R²²: gleich oder verschieden sind und Wasserstoff, Phenyl oder geradkettiges oder verzweigtes Alkyl mit bis zu 3 Kohlenstoffatomen bedeuten,
- R⁴: Wasserstoff oder geradkettiges oder verzweigtes Alkyl mit bis zu 3 Kohlenstoffatomen bedeutet,
- R⁵ und R⁸: gleich oder verschieden sind und Wasserstoff oder Methyl bedeuten,
- R⁶: Wasserstoff oder geradkettiges oder verzweigtes Alkyl mit bis zu 3 Kohlenstoffatomen bedeutet, das gegebenenfalls durch Hydroxy substituiert ist,
- R⁷: geradkettiges oder verzweigtes Alkyl mit 2 bis 6 Kohlenstoffatomen bedeutet, das durch eine Gruppe der Formel -NR²³R²⁴ substituiert ist,
worin
- R²³ und R²⁴: gleich oder verschieden sind und Wasserstoff oder geradkettiges oder verzweigtes Alkyl mit bis zu 3 Kohlenstoffatomen bedeuten, das gegebenenfalls durch Hydroxy substituiert ist,
oder durch Phenyl substituiert ist, das seinerseits durch die Gruppe der Formel -SO₂-R²⁵R²⁶ substituiert ist,
worin
- R²⁵ und R²⁶: die oben angegebene Bedeutung von R⁹ und R¹⁰ haben,
- D: für Wasserstoff oder für eine Gruppe der Formel -SO₂-NR²⁷R²⁸ steht,
worin
- R²⁷ und R²⁸: gleich oder verschieden sind und die oben angegebene Bedeutung von R⁹ und R¹⁰ haben und mit dieser gleich oder verschieden sind,
- E: für geradkettiges oder verzweigtes Alkyl mit bis zu 5 Kohlenstoffatomen steht,
und deren Tautomere und Salze.

Außerdem wurde ein Verfahren zur Herstellung der erfindungsgemäßen Verbindungen der allgemeinen Formel (I) gefunden, dadurch gekennzeichnet, daß man zunächst Verbindungen der allgemeinen Formel (II) 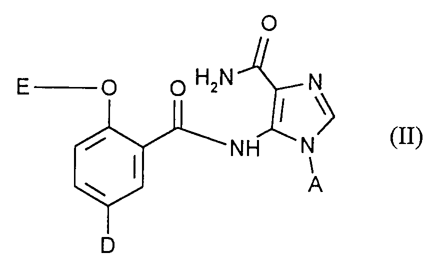
in welcher
- D, E und A: die oben angegebene Bedeutung haben,
in inerten Lösemitteln und in Anwesenheit einer Base cyclisiert,
und im Fall D = H zunächst durch Umsetzung mit Chlorsulfonsäure die entsprechenden Sulfonsäurechloride herstellt und anschließend mit Aminen (NR²⁵R²⁶) die entsprechenden Sulfonamide herstellt,
und die Substituenten R¹ - R⁸ nach üblichen Methoden, wie beispielsweise Alkylierung, Acylierung, Aminierung, Oxidation oder Azidaustausch einführt bzw. derivatisiert.

Das erfindungsgemäße Verfahren kann durch folgende Formelschemata beispielhaft erläutert werden: 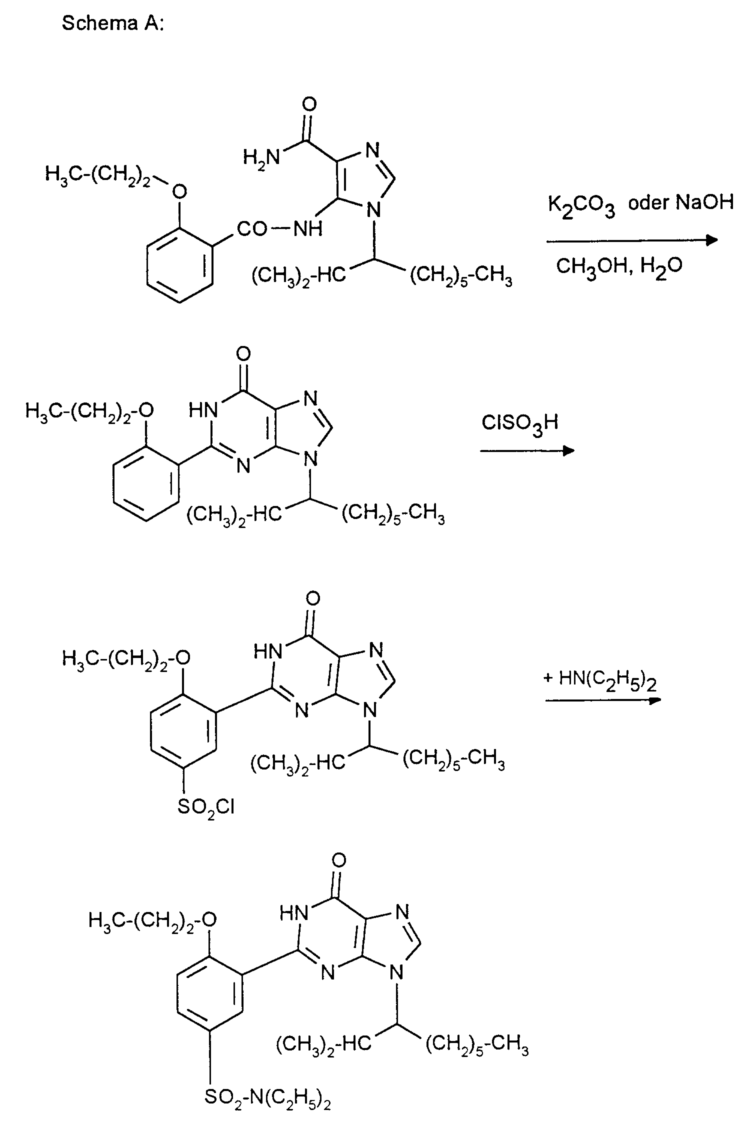 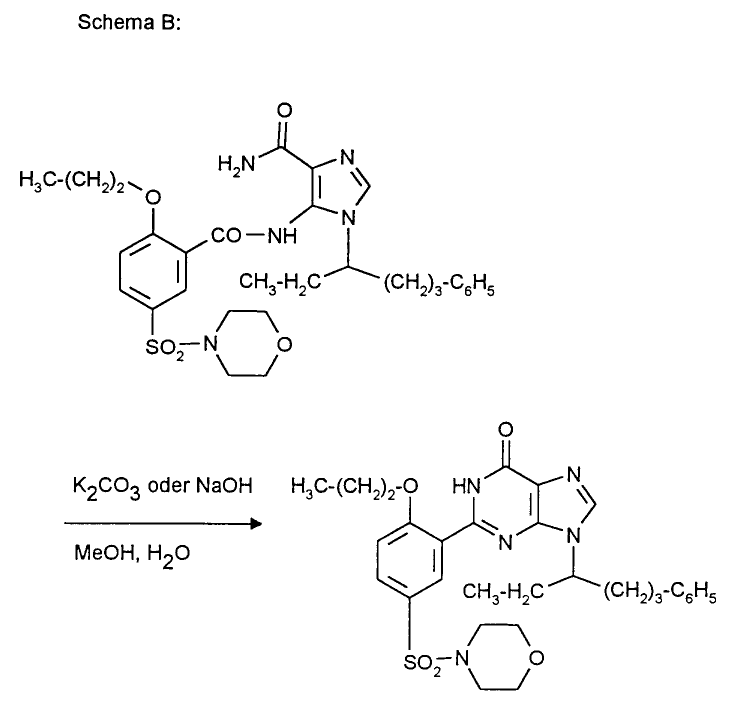
Für das Verfahren eignen sich inerte organische Lösemittel, die sich unter den Reaktionsbedingungen nicht verändern. Hierzu gehören bevorzugt Ether wie beispielsweise Diethylether, Dioxan, Tetrahydrofuran, Glykolmono- oder dimethylether, Essigester, Toluol, Acetonitril, Hexamethylphosphorsäuretriamid, Pyridin und Aceton. Selbstverständlich ist es möglich, Gemische der Lösemittel einzusetzen. Besonders bevorzugt sind Tetrahydrofuran, Toluol oder Pyridin.

Als Lösemittel für die Cyclisierung eignen sich die üblichen organischen Lösemittel. Hierzu gehören bevorzugt Alkohole wie Methanol, Ethanol, Propanol, Isopropanol oder Butanol, oder Ether wie Tetrahydrofuran oder Dioxan, oder Dimethylformamid oder Dimethylsulfoxid. Besonders bevorzugt werden Alkohole wie Methanol, Ethanol, Propanol oder Isopropanol verwendet. Ebenso ist es möglich, Gemische der genannten Lösemittel einzusetzen.

Als Basen eignen sich für die Cyclisierung die üblichen anorganischen Basen. Hierzu gehören bevorzugt Alkalihydroxide oder Erdalkalihydroxide wie beispielsweise Natriumhydroxid, Kaliumhydroxid oder Bariumhydroxid, oder Alkalicarbonate wie Natrium- oder Kaliumcarbonat oder Natriumhydrogencarbonat, oder Alkalialkoholate wie Natriummethanolat, Natriumethanolat, Kaliummethanolat, Kaliumethanolat oder Kalium-tert.butanolat. Besonders bevorzugt sind Kaliumcarbonat, und Natriumhydroxid.

Bei der Durchführung der Cyclisierung wird die Base im allgemeinen in einer Menge von 2 bis 6 mol, bevorzugt von 3 bis 5 mol bezogen auf 1 mol der Verbindungen der Formel (II), eingesetzt.

Die Cyclisierung wird im allgemeinen in einem Temperaturbereich von 0°C bis 160°C, bevorzugt bei der Siedetemperatur des jeweiligen Lösemittels durchgeführt.

Die Cyclisierung wird im allgemeinen bei Normaldruck durchgeführt. Es ist aber auch möglich, das Verfahren bei Überdruck oder bei Unterdruck durchzuführen (z.B. in einem Bereich von 0,5 bis 5 bar).

Die Chlorsulfonierung erfolgt entweder ohne Lösemittel oder in Anwesenheit eines der oben aufgeführten inerten Lösemittel.

Die Herstellung der Sulfonamide erfolgt im allgemeinen in einem der oben aufgeführten Lösemittel, vorzugsweise in Tetrahydrofuran oder Dichlormethan.

Die Chlorsulfonierung und die Amidierung erfolgen im allgemeinen in einem Temperaturbereich von -20 C bis +80 C, vorzugsweise von -10 C bis +30 C und Normaldruck.

Als Basen eignen sich dafür neben den oben aufgeführten Basen vorzugsweise in einigen Fällen Triethylamin und/oder Dimethylaminopyridin, DBU oder DABCO. Es ist ebenso möglich, das eingesetzte Amin im Überschuß einzusetzen.

Die Base wird in einer Menge von 0,5 mol bis 10 mol, bevorzugt von 1 mol bis 3 mol, bezogen auf 1 mol der entsprechenden Chlorsulfonsäure, eingesetzt.

Die Alkylierung erfolgt im allgemeinen mit Alkylierungsmitteln wie beispielsweise (C₁-C₁₀)-Alkylhalogeniden, Sulfonsäureestern oder substituierten oder unsubstituierten (C₁-C₁₀)-Dialkyl- oder (C₁-C₁₀)Diarylsulfonate, vorzugsweise Methyliodid oder Dimethylsulfat.

Als Lösemittel für die Alkylierung eignen sich ebenfalls übliche organische Lösemittel, die sich unter den Reaktionsbedingungen nicht verändern. Hierzu gehören bevorzugt Ether wie Diethylether, Dioxan, Tetrahydrofuran, Glykoldimethylether, oder Kohlenwasserstoffe wie Benzol, Toluol, Xylol, Hexan, Cyclohexan oder Erdölfrakionen, oder Halogenkohlenwasserstoffe wie Dichlormethan, Trichlormethan, Tetrachlormethan, Dichlorethylen, Trichlorethylen oder Chlorbenzol, oder Essigester, Dimethylformamid, Hexamethylphosphorsäuretriamid, Acetonitril oder Aceton. Ebenso ist es möglich, Gemische der genannten Lösemittel zu verwenden. Bevorzugt ist Dichlormethan.

Die Alkylierung wird in den oben aufgeführten Lösemitteln bei Temperaturen von 0°C bis +150°C, vorzugsweise bei Raumtemperatur bis +100°C, bei Normaldruck durchgeführt.

Die Abspaltung der Aminoschutzgruppen erfolgt in an sich bekannter Weise unter sauren oder basischen Bedingungen, oder reduktiv durch katalytische Hydrierung beispielsweise mit Pd/C in organischen Lösemitteln wie Ethern, z.B. Tetrahydrofuran oder Dioxan, oder Alkoholen z.B. Methanol, Ethanol oder Isopropanol.

Die Abspaltung der Hydroxyschutzgruppe aus den entsprechenden Estern erfolgt nach üblichen hydrolytischen Methoden.

Die Umsetzung mit Alkylsulfonsäurechloriden erfolgt, ausgehend von den entsprechenden freien Hydroxyverbindungen, in einem der oben aufgeführten Lösemittel und einer der Basen, vorzugsweise mit Dichlormethan und Triethylamin in einem Temperaturbereich von -20C bis +20°C, vorzugsweise 0°C und Normaldruck.

Die Einführung des Azidrestes erfolgt im allgemeinen durch Umsetzung der entsprechenden Alkylsulfonyloxy substituierten Verbindungen mit Natriumazid in einem der oben aufgeführten Lösemittel, vorzugsweise Dimethylformamid, in einem Temperaturbereich von 50°C bis +120°C, vorzugsweise 100°C und Normaldruck.

Die Reduktion der Azido-substituierten Verbindungen zu den entsprechenden freien Aminen erfolgt im allgemeinen durch Hydrierung in einem der oben aufgeführten Alkoholen, vorzugsweise Methanol, mit Wasserstoff in Anwesenheit eines Palladiumkatalysators, vorzugsweise Pd/C im einem Temperaturbereich von 0°C bis +50°C, vorzugsweise 25°C.

Die Hydrierung erfolgt im allgemeinen bei Normaldruck.

Die Acylierungen erfolgen ausgehend von dem entsprechenden freien Amino bzw. Hydroxyverbindungen in einem der oben aufgeführten Lösemitteln, vorzugsweise Dichlormethan und in Anwesenheit einer der oben aufgeführten Basen, vorzugsweise Triethylamin mit Zusatz von Dimethylaminopyridn (DMAP) in einem Temperaturbereich von 0°C bis +80°C, vorzugsweise bei +20°C bis +40°C und Normaldruck.

Die Ketone werden ausgehend von den entsprechenden Hydroxyverbindungen nach bekannten Methoden (Swern-Oxidation) hergestellt.

Die Oximbildung erfolgt im allgemeinen durch Umsetzung des entsprechenden Ketons mit Hydroxylaminen in einen der oben aufgeführten Alkoholen, vorzugsweise Methanol, bei Rückflußtemperatur und Normaldruck.

Die enantiomerenreinen Verbindungen sind nach üblichen Methoden, beispielsweise durch Chromatographie der racemischen Verbindungen der allgemeinen Formel (I) auf chiralen Phasen zugänglich.

Die Verbindungen der allgemeinen Formel (II) sind teilweise bekannt oder neu und können hergestellt werden, indem man Verbindungen der allgemeinen Formel (III) 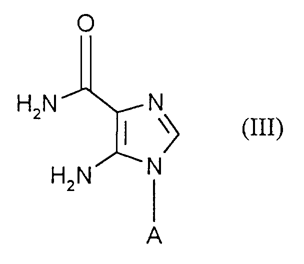
in welcher
- A: die oben angegebene Bedeutung hat,
durch Umsetzung mit 2-n-Alkoxybenzoesäurechloriden der allgemeinen Formel (IV) 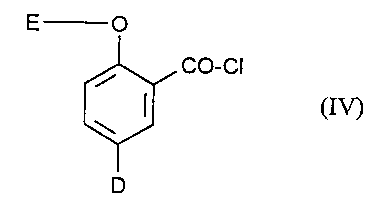
in welcher
- D und E: die oben angegebene Bedeutung haben,
in inerten Lösemitteln und in Anwesenheit einer Base, umsetzt.

Als Lösungmittel eignen sich die oben aufgeführten Lösemittel, wobei Toluol und Tetrahydrofuran bevorzugt sind.

Als Basen eignen sich im allgemeinen Alkalihydride oder -alkokolate, wie beispielsweise Natriumhydrid oder Kalium-tert.-butylat, oder cyclische Amine, wie beispielsweise Piperidin, Pyridin, Dimethylaminopyridin oder C₁-C₄-Alkylamine, wie beispielsweise Triethylamin. Bevorzugt sind Natriumhydrid, Pyridin und Dimethylaminopyridin.

Die Base wird im allgemeinen in einer Menge von 1 mol bis 4 mol, bevorzugt von 1,2 mol bis 3 mol jeweils bezogen auf 1 mol der Verbindungen der allgemeinen Formel (III) eingesetzt.

Die Reaktionstemperatur kann im allemeinen in einem größeren Bereich varriert werden. Im allgemeinen arbeitet man in einem Bereich von -20°C bis 200°C, bevorzugt von 0°C bis 25°C.

In einer Variante wird die Umsetzung in Pyridin, dem eine katalytische Menge DMAP zugesetzt wird, durchgeführt. Gegebenenfalls kann noch Toluol zugeführt werden.

Die Verbindungen der allgemeinen Formel (IV) sind an sich bekannt.

Die Verbindungen der allgemeinen Formel (III) sind größtenteils neu und können beispielsweise hergestellt werden, indem man
2-Amino-2-cyanoacetamid der Formel (V) 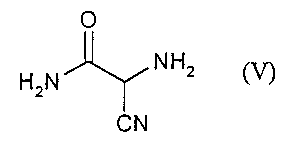
mit Verbindungen der allgemeinen Formel (VI) 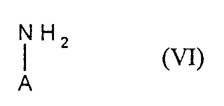
in welcher
- A: die oben angegebene Bedeutung hat,
in inerten Lösemitteln, in Anwesenheit von Triethylorthoformiat umsetzt.

Als Lösemittel für die einzelnen Schritte dei Verfahren eignen sich die üblichen organischen Lösemittel, die sich unter den Reaktionsbedingungen nicht verändern. Hierzu gehören bevorzugt Ether wie Diethylether, Dioxan, Tetrahydrofuran, Glykoldimethylether, oder Kohlenwasserstoffe wie Benzol, Toluol, Xylol, Hexan, Cyclohexan oder Erdölfrakionen, oder Halogenkohlenwasserstoffe wie Dichlormethan, Trichlormethan, Tetrachlormethan, Dichlorethylen, Trichlorethylen oder Chlorbenzol, oder Essigester, Dimethylformamid, Hexamethylphosphorsäuretriamid, Acetonitril, Aceton oder Dimethoxyethan. Ebenso ist es möglich, Gemische der genannten Lösemittel zu verwenden. Besonders bevorzugt ist Acetonitril.

Das erfindungsgemäße Verfahren wird im allgemeinen in einem Temperaturbereich von 0°C bis +180°C, bevorzugt von +30°C bis +150°C durchgeführt.

Die erfindungsgemäßen Verfahrenschritte werden im allgemeinen bei Normaldruck durchgeführt. Es ist aber auch möglich, bei Überdruck oder bei Unterdruck zu arbeiten (z.B. in einem Bereich von 0,5 bis 5 bar).

Die Verbindung der Formel (V) ist bekannt [vgl. Logemann, G. Shaw, Chemistry and Industry, 1980 (13), 541-542].

Die Amine der allgemeinen Formel (VI) sind teilweise bekannt oder neu und können dann nach bekannten Methoden hergestellt werden [vgl. L.R. Krepski et al., Synthesis, 1986, 301-303].

Die erfindungsgemäßen Verbindungen der allgemeinen Formel (I) zeigen ein nicht vorhersehbares, wertvolles pharmakologisches Wirkspektrum.

Sie inhibieren entweder eine oder mehrere der c-GMP metabolisierenden Phosphodiesterasen (PDE I, PDE II und PDE V). Dies führt zu einem differenzierten Anstieg von c-GMP. Eine Erhöhung des c-GMP-Spiegels, kann zu einer antithrombotischen, vasodilatorischen, antiarrhythmischen und/oder antientzündlichen Wirkung führen. Die Selektivität wird von der Verteilung der Isoenzyme im Gewebe mitbestimmt.

Außerdem verstärken die erfindungsgemäßen Verbindungen die Wirkung von Substanzen, wie beispielsweise EDRF (Endothelium derived relaxing factor) und ANP (atrial natriuretic peptide), die den cGMP-Spiegel steigern.

Sie können daher in Arzneimitteln zur Behandlung von entzündlichen Erkrankungen wie beispielsweise Asthma, entzündlichen Dermatosen, zur Behandlung des Bluthochdrucks, stabiler und instabiler Angina, peripheren und kardialen Gefäßerkrankungen, von Arrhythmien, zur Behandlung von thromboembolischen Erkrankungen und Ischämien wie Myokardinfarkt, Hirnschlag, transistorisch und ischämische Attacken, Angina pectoris, periphere Durchblutungsstörungen, Verhinderung von Restenosen wie nach Thrombolysetherapien, percutan transluminalen Angioplastien (PTA) und Bypass, percutan transluminalen Koronarangioplastien (PTCA), Bypass, septischem Schock und Krankheiten des Urogenitalsystems wie beispielsweise Prostatahypertrophy, Impotenz und Inkontinenz eingesetzt werden.

### Aktivität der Phosphordiesterasen (PDE's)

Die c-GMP stimulierbare PDE II, die c-GMP hemmbare PDE III und die cAMP spezifische PDE IV wurden entweder aus Schweine- oder Rinderherzmyokard isoliert. Die Ca-Cal-modulinstimulierbare PDE I wurde aus Schweineaorta oder Schweinegehirn isoliert. Die c-GMP spezifische PDE V wurde aus Schweinedünndarm, Schweineaorta und/oder humanen Blutplättchen gewonnen. Die Reinigung erfolgte durch Anionenaustauschchromatographie an MonoQ^{R} Pharmacia im wesentlichen nach der Methode von M. Hoey and Miles D. Houslay, Biochemical Pharmacology, Vol. 40, 193-202 (1990).

Die Bestimmung der Enzymaktivität erfolgt in einem Testansatz von 100 µl in 20 mM Tris/HCl-Puffer pH 7,5 der 5 mM MgCl₂, 0,1 mg/ml Rinderserumalbumin und entweder 800 Bq ³HcAMP oder ³HcGMP enthält. Die Endkonzentration der entsprechenden Nucleotide ist 10⁻⁶ mol/l. Die Reaktion wird durch Zugabe des Enzyms gestartet, die Enzymmenge ist so bemessen, daß während der Inkubationszeit von 30 min ca 50% des Substrates umgesetzt werden. Um die cGMP stimulierbare PDE II zu testen, wird als Substrat ³HcAMP verwendet und dem Ansatz 10⁻⁶ Mol/l nicht markiertes cGMP zugesetzt. Um die Ca-Calmodulinabhängige PDE I zu testen, werden dem Reaktionsansatz noch CaCl₂ 1 µM und Calmodulin 0,1 µM zugesetzt. Die Reaktion wird durch Zugabe von 100 µl Acetonitril, das 1 mM cAMP und 1 mM AMP enthält, gestoppt. 100 µl des Reaktionsansatzes werden auf der HPLC getrennt und die Spaltprodukte "Online" mit einem Durchflußscintillationszähler quantitativ bestimmt. Es wird die Substanzkonzentration gemessen, bei der die Reaktionsgeschwindigkeit um 50% vermindert ist.

### Inhibition der Phosphodiesterasen in vitro

| Bsp.-Nr. | PDE I IC₅₀ [µM] | PDE II IC₅₀ [µM] | PDE V IC₅₀ [µM] |
|---|---|---|---|
| 3 | 50 | 6 | 0,1 |
| 4 | 2 | 1 | 5 |
| 5 | 1 | 0,5 | 2 |
| 11 | 0,5 | 0,3 | 0,5 |
| 37 | 2 | 2 | 1 |
| 57 | 0,3 | 0,3 | 0,1 |

Die antihypertensive Aktivität wurde nach intravenöser Applikation an SHR-Ratten gemessen.

Zur Bestimmung der cyclischen Nucleotide wurden Herz- und Aortengewebe entnommen und unmittelbar tiefgefroren. Die Proben wurden unter flüssigen N₂ pulverisiert, mit 70% Ethanol extrahiert und der Gehalt am cGMP und cAMP mit kommerziellen Radioimmunoassays (Amersham) bestimmt.

Die erektionsauslösende Wirkung wurde am narkotisierten Kaninchen gemessen. (C.G. Stief et al. World Journal Urology 1990, S. 233-236).

Die Substanzen wurden in Dosierungen von 0,1 bis 10 mg/kg entweder direkt in den Corpus cavernosum, intraduodenal, rektal, oral, transdermal oder intravenös appliziert.

Die neuen Wirkstoffe können in bekannter Weise in die üblichen Formulierungen überführt werden, wie Tabletten, Dragees, Pillen, Granulate, Aerosole, Sirupe, Emulsionen, Suspensionen und Lösungen, unter Verwendung inerter, nicht toxischer, pharmazeutisch geeigneter Trägerstoffe oder Lösungsmittel. Hierbei soll die therapeutisch wirksame Verbindung jeweils in einer Konzentration von etwa 0,5 bis 90-Gew.-% der Gesamtmischung vorhanden sein, d.h. in Mengen, die ausreichend sind, um den angegebenen Dosierungsspielraum zu erreichen.

Die Formulierungen werden beispielsweise hergestellt durch Verstrecken der Wirkstoffe mit Lösungsmitteln und/oder Trägerstoffen, gegebenenfalls unter Verwendung von Emulgiermitteln und/oder Dispergiermitteln, wobei z.B. im Fall der Benutzung von Wasser als Verdünnungsmittel gegebenenfalls organische Lösungsmittel als Hilfslösungsmittel verwendet werden können.

Die Applikation erfolgt in üblicher Weise, vorzugsweise oral, parenteral, transdermal, perlingual oder intravenös.

Im allgemeinen hat es sich als vorteilhaft erwiesen, bei intravenöser Applikation Mengen von etwa 0,01 bis 10 mg/kg, vorzugsweise etwa 0,1 bis 10 mg/kg Körpergewicht zur Erzielung wirksamer Ergebnisse zu verabreichen.

Trotzdem kann es gegebenenfalls erforderlich sein, von den genannten Mengen abzuweichen, und zwar in Abhängigkeit vom Körpergewicht bzw. der Art des Applikationsweges, vom individuellen Verhalten gegenüber dem Medikament, der Art von dessen Formulierung und dem Zeitpunkt bzw. Intervall, zu welchen die Verabreichung erfolgt. So kann es in einigen Fällen ausreichend sein, mit weniger als der vorgenannten Mindestmenge auszukommen, während in anderen Fällen die genannte obere Grenze überschritten werden muß. Im Falle der Applikation größerer Mengen kann es empfehlenswert sein, diese in mehreren Einzelgaben über den Tag zu verteilen.

### Ausgangsverbindungen

Allgemeine Arbeitsvorschrift zur Herstellung von 1-substituierten 5-(2-n-Alkoxybenzoylamino)-imidazol-4-carboxamiden der Formel (IV)

### Methode A:

10 mmol 1-substituiertes 5-Amino-imidazol-4-carboxamid und 15 mmol NaH (enthält einer der oben angegebenen Reste R¹, R², R³, R⁴, R⁵, R⁶, R⁷ oder R⁸ eine Hydroxygruppe werden 30 mmol NaH eingesetzt) werden in 50 ml abs. THF 3 h bei 20°C gerührt (bei schwerlöslichen Imidazolen wird bis zu 12 h refluxiert). Bei 20°C werden 10 mmol 2-n-Alkoxybenzoesäurechlorid (bzw. 20 mmol bei Vorhandensein einer Hydroxygruppe) in 25 ml abs. THF zugetropft und über Nacht bei Raumtemperatur gerührt. Es wird eingedampft, der Rückstand in Essigsäureethylester aufgenommen und mit Wasser ausgeschüttelt. Die organische Phase wird über Na₂SO₄ getrocknet, eingedampft und der Rückstand durch Umkristallisation oder Flashchromatographie gereinigt.

Enthält das 5-Amino-imidazol-4-carboxamid in den Resten R¹, R², R³, R⁴, R⁵, R⁶, R⁷ oder R⁸ eine freie Hydroxygruppe, so liegt diese als 2-n-Alkoxybenzoesäureester vor, der nach bekannter Methode (1 n NaOH, CH₃OH) verseift werden kann. Es ist jedoch auch möglich, die Ester direkt mit NaOH als Base zu den Purinonen zu cyclisieren, wobei der Ester ebenfalls hydrolysiert wird.

### Methode B:

10 mmol 1-substituiertes 5-Amino-imidazol-4-carboxamid und 50 mg DMAP werden in 20 ml trockenem Pyridin bei Raumtemperatur vorgelegt. Dazu wird eine Lösung von 10 mmol n-Alkoxybenzoesäurechlorid in 10 ml abs. Toluol zugetropft und bei Raumtemperatur gerührt bis im DC die Umsetzung beendet ist (30 Minuten bis 16 Stunden). Der Niederschlag wird abfiltriert und das Lösemittel im Vakuum abrotiert. Der Rückstand wird in 30 ml Methylenchlorid aufgenommen und mit 30 ml 1 n HCl und 30 ml H₂ O gewaschen. Nach Trocknen über Na₂SO₄ wird im Vakuum eingedampft und der Rückstand durch Flashchromatographie oder Umkristallisation gereinigt.

Nach diesen Vorschriften werden die in der Tabelle I aufgeführten 1-substituierten 5-(2-n-Alkoxybenzoylamino)-imidazol-5-carboxamide hergestellt:

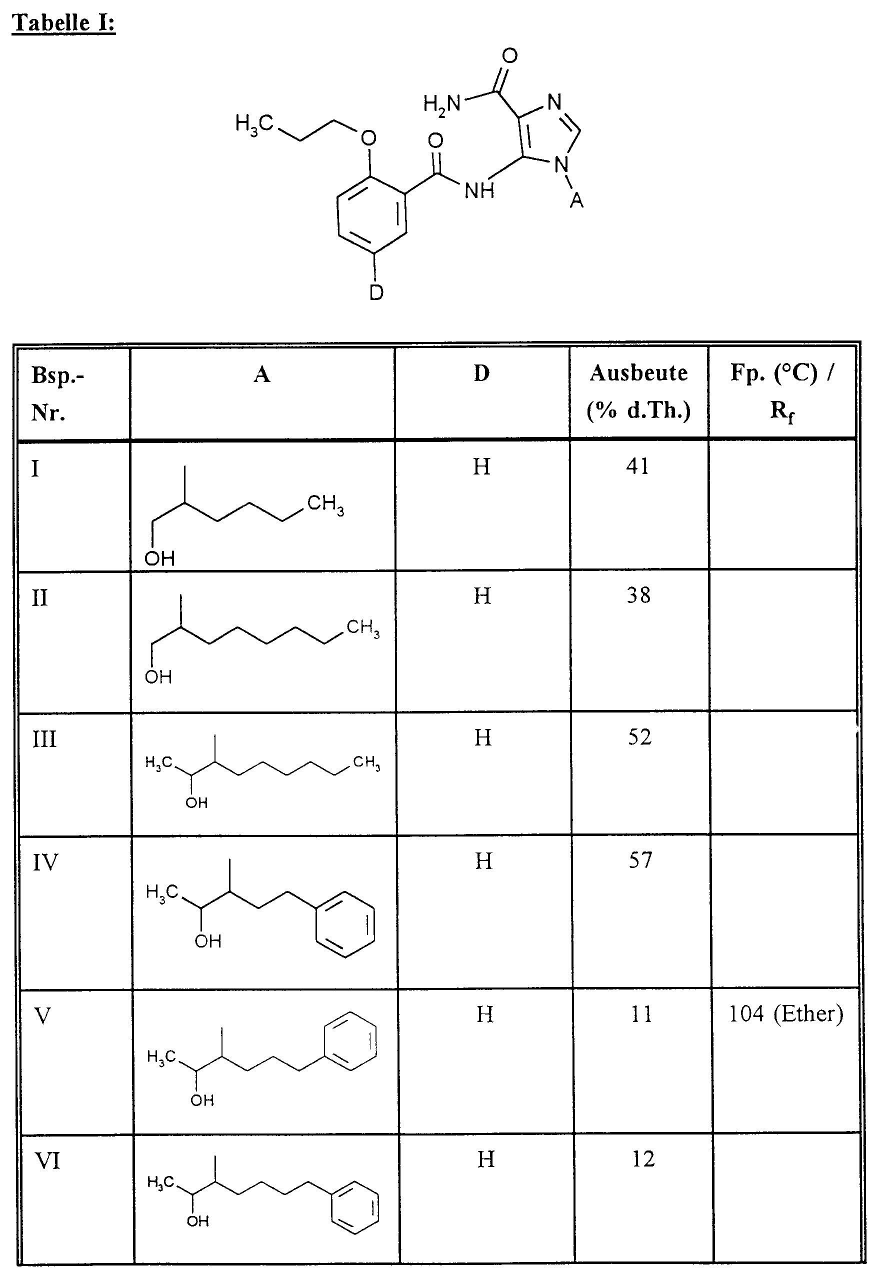

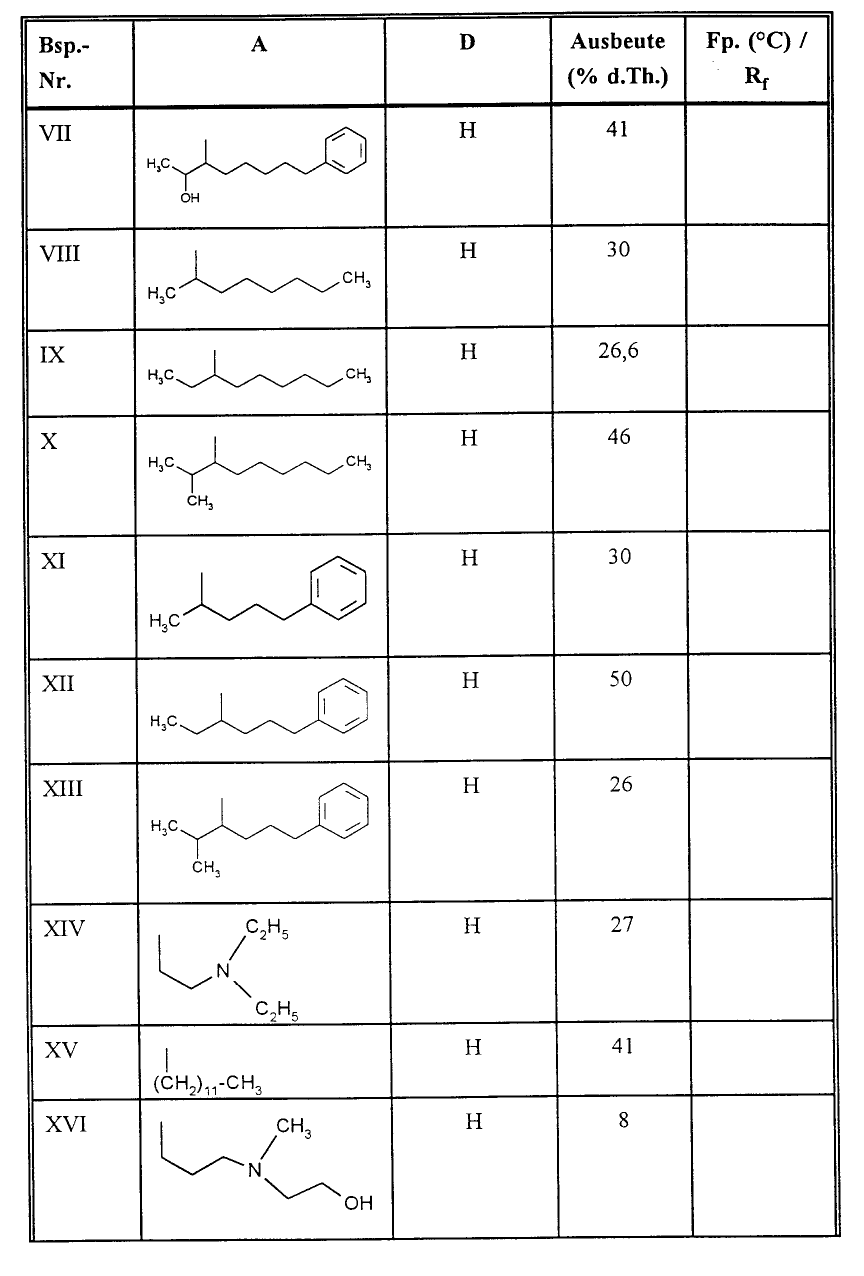

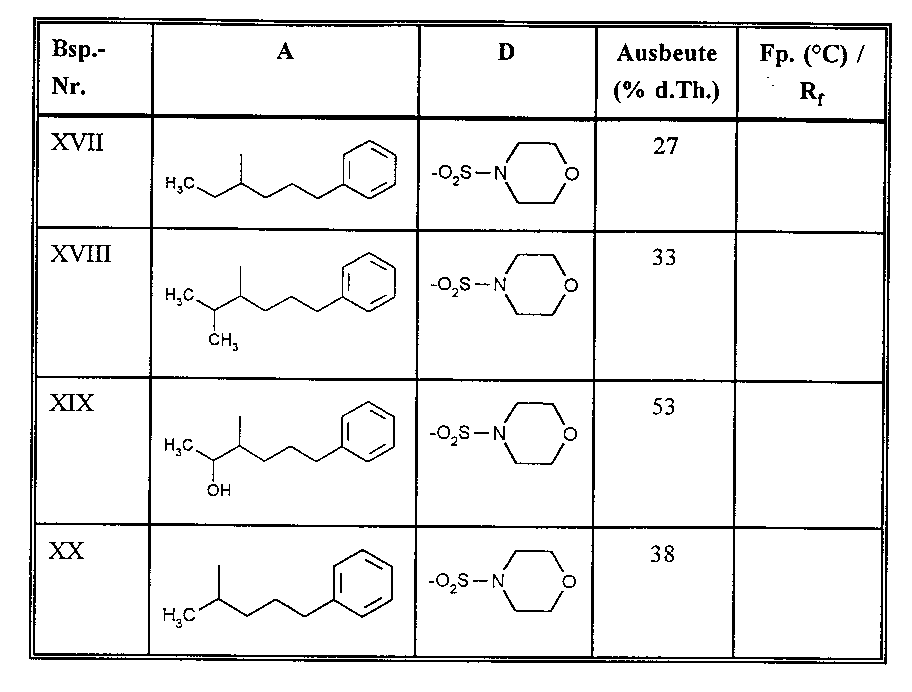

### Herstellungsbeispiele

Allgemeine Arbeitsvorschrift für 9-substituierte 2-(2-n-Alkoxyphenyl)purin-6-one der allgemeinen Formel (I) 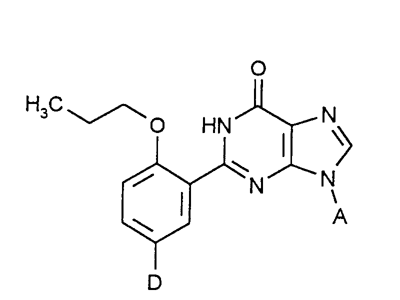
10 mmol des 1-substituierten 5-(2-n-Alkoxybenzoylamino)-imidazol-4-carboxamids (IV) und 40 mmol K₂CO₃ werden in einem Gemisch von 100ml Ethanol und 50 ml Wasser über Nacht refluxiert. Das Lösemittel wird im Vakuum abdestilliert, der Rückstand in Essigsäureethylester aufgenommen und zweimal mit Wasser ausgeschüttelt. Nach Trocknen der organischen Phase mit Natriumsulfat wird eingedampft, und der Rückstand durch Umkristallisation oder Flashchromatographie gereinigt.

Nach dieser Vorschrift werden die in der Tabelle II aufgeführten Verbindungen hergestellt:

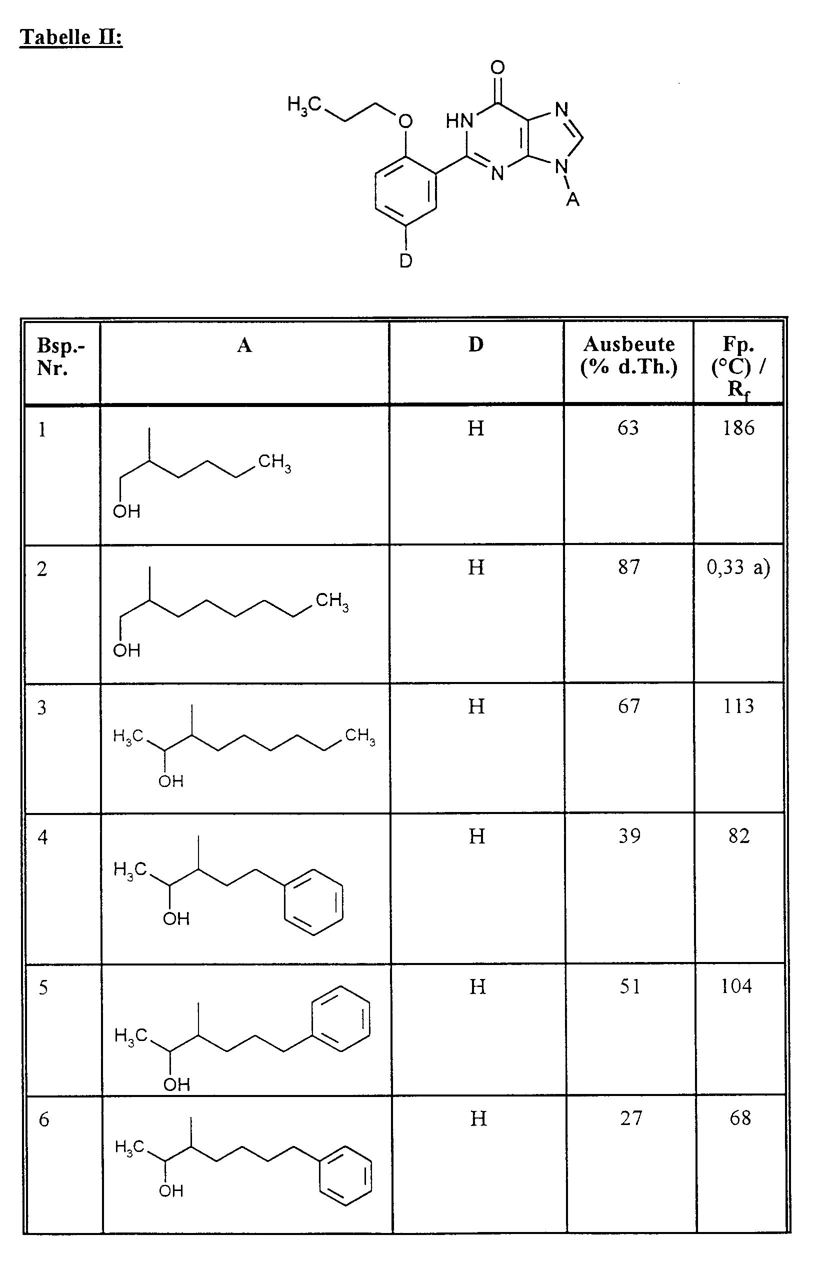

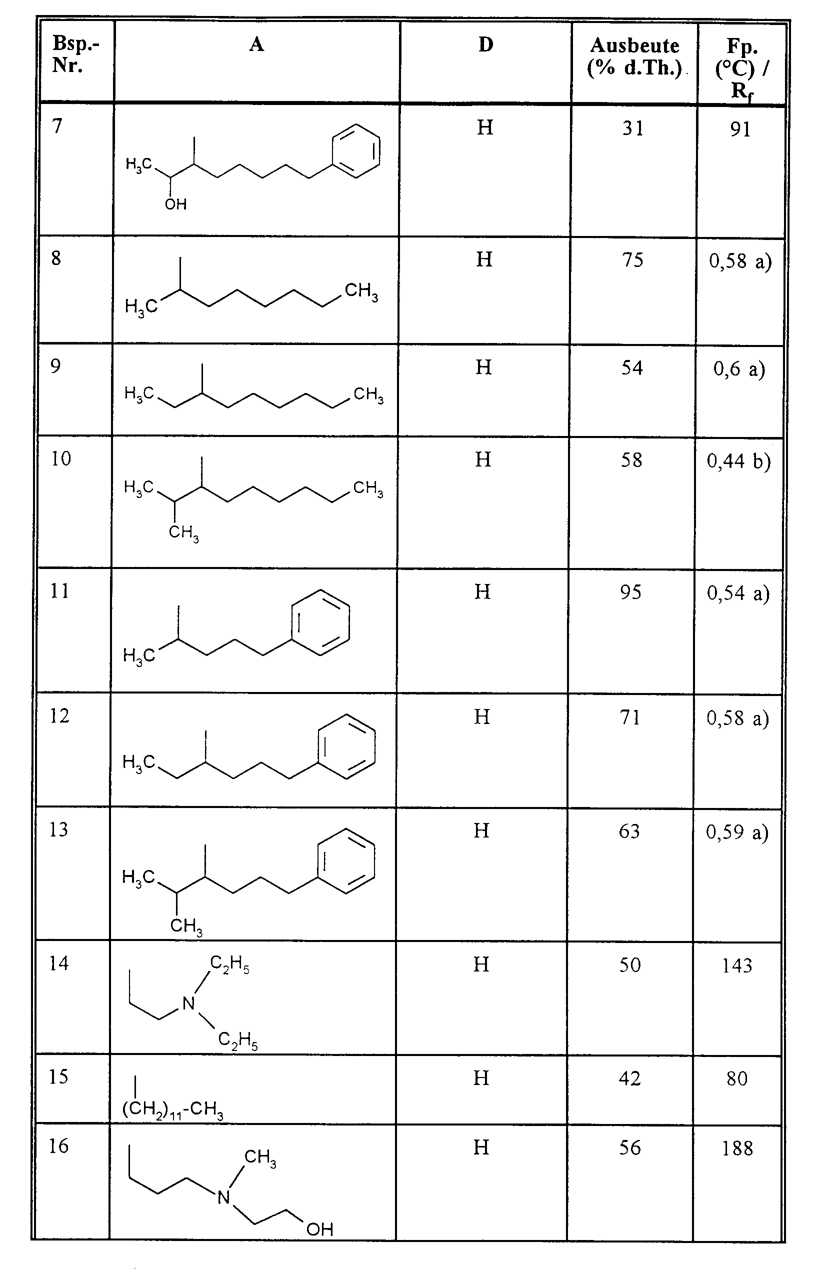

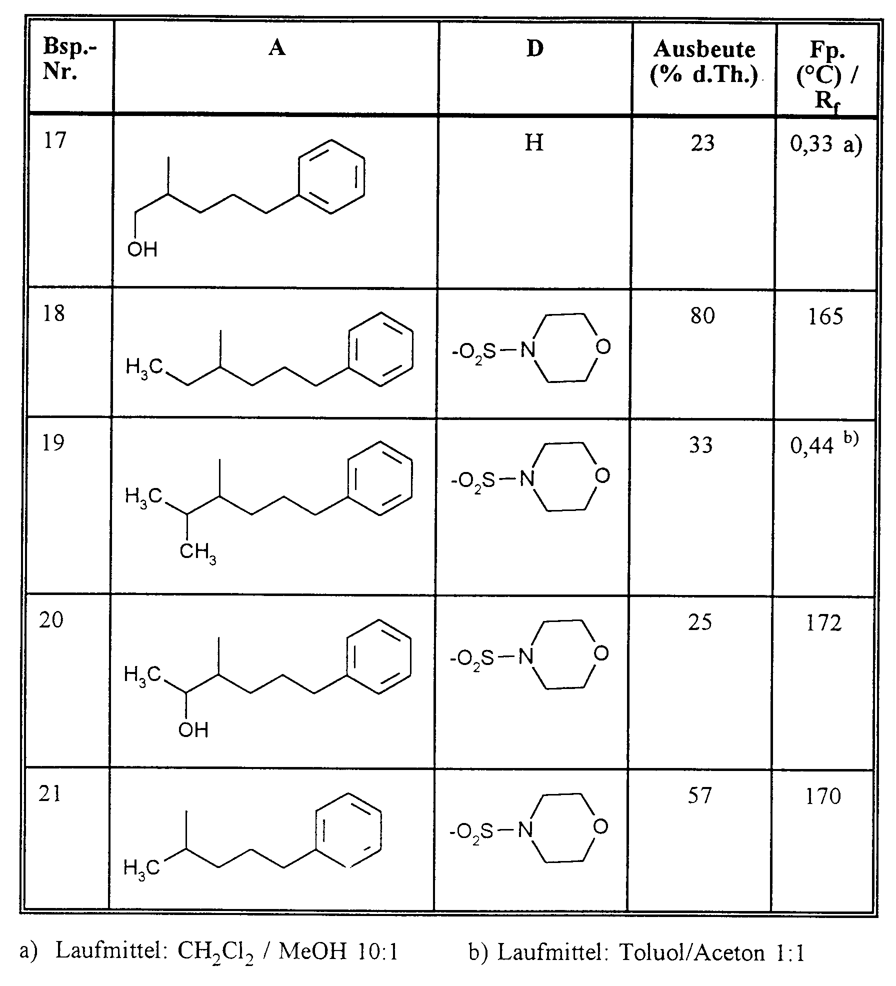

### Beispiel 22

9-(2-Methansulfonyloxy-3-nonyl)-2-(2-n-propoxy-phenyl)-purin-6-on 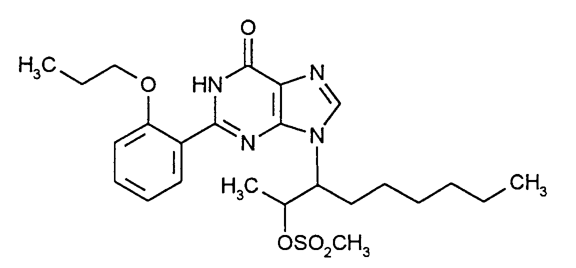
412 mg (1mmol) 9-(2-Hydroxy-3-nonyl)-2-(2-n-propoxyphenyl)-purin-6-on (Beispiel 3) werden in 10 ml CH₂Cl₂ auf 0°C gekühlt. Nach Zugabe von 0,5 ml Triethylamin werden 138 mg (1,2 mmol) Methansulfonsäurechlorid in 2 ml CH₂Cl₂ zugetropft und 30 min nachgerührt. Es wird auf 20 ml Eiswasser gegossen, die organische Phase abgetrennt und die wäßrige Phase noch einmal mit 20 ml CH₂Cl₂ extrahiert. Die vereinigten CH₂Cl₂-Phasen werden über Na₂SO₄ getrocknet, im Vakuum eingedampft und der ölige Rückstand durch Verreiben mit Ether kristallisiert.
Fp.: 158°C
Ausbeute: 380 mg (81%)

### Beispiel 23

9-(2-Methansulfonyloxy-5-phenyl-3-pentyl)-2-(2-n-propoxyphenyl)-purin-6-on 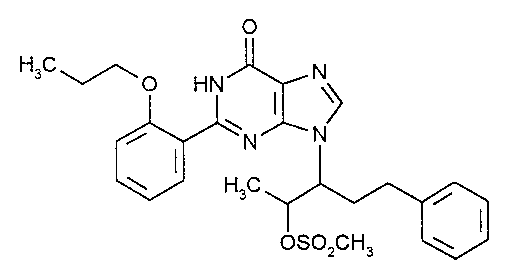
Die Titelverbindung wird in Analogie zur Vorschrift des Beispiels 22, ausgehend von 9-(2-Hydroxy-5-phenyl-3methyl)-2-(2-n-propoxyphenyl)purin-6-on (Beispiel 4) hergestellt.
R_{f} = 0,52 (CH₂Cl₂/CH₃OH 10:1)
Ausbeute: 95,3%

### Beispiel 24

9-(2-Methansulfonyloxy-6-phenyl-3-hexyl)-2-(2-n-propoxyphenyl)-purin-6-on 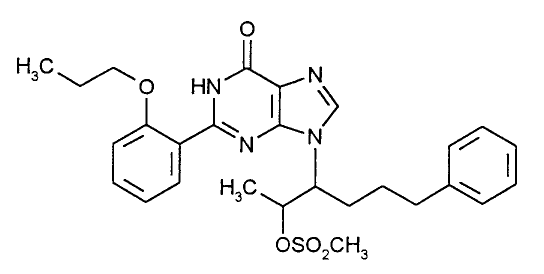
Die Titelverbindung wird in Analogie zur Vorschrift des Beispiels 22, ausgehend von 9-(2-Hydroxy-6-phenyl-3-hexyl)-2-(2-n-propoxyphenyl)-purin-6-on (Beispiel 5) hergestellt.
R_{f} = 0,52 (CH₂Cl₂/CH₃OH 10:1)
Ausbeute: 82,7%

### Beispiel 25

9-(2-Methansulfonyloxy-7-phenyl-3-heptyl)-2-(2-n-propoxyphenyl)-purin-6-on 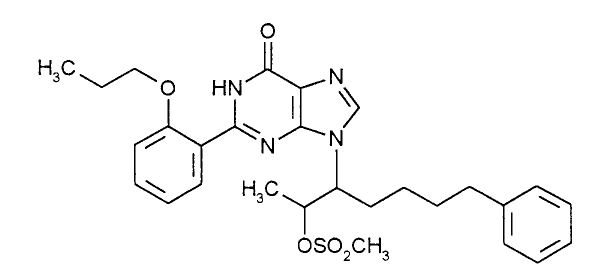
Die Titelverbindung wird in Analogie zur Vorschrift des Beispiels 22, ausgehend von 9-(2-Hydroxy-7-phenyl-3-heptyl)-2-(2-n-propoxyphenyl)-purin-6-on (Beispiel 6) hergestellt.
R_{f} = 0,55 (CH₂Cl₂/CH₃OH 10:1)
Ausbeute: 90,3%

### Beispiel 26

9-(2-Methansulfonyloxy-8-phenyl-3-octyl)-2-(2-n-propoxyphenyl)-purin-6-on 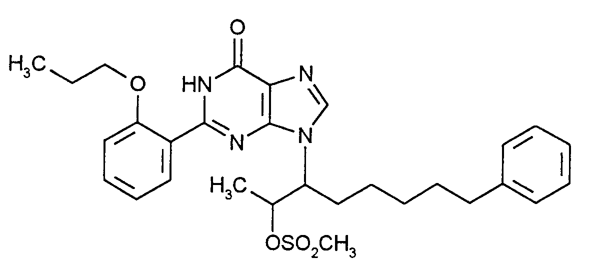
Die Titelverbindung wird in Analogie zur Vorschrift des Beispiels 22, ausgehend von 9-(2-Hydroxy-8-phenyl-3-octyl)-2-(2-n-propoxyphenyl)-purin-7-on (Beispiel 7) hergestellt.
R_{f} = 0,58 (CH₂Cl₂ / CH₃OH 10:1)
Ausbeute: 89%

### Beispiel 27

9-(1-Methansulfonyloxy-5-phenyl-2-pentyl)-2-(2-n-propoxyphenyl)-purin-6-on 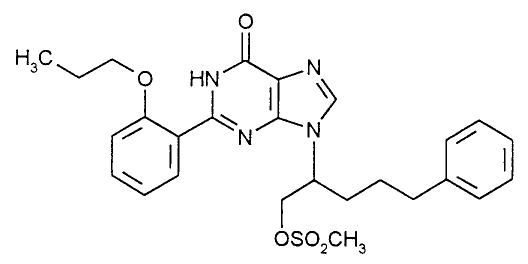
Die Titelverbindung wird in Analogie zur Vorschrift des Beispiels 22 ausgehend von 9-(1-Hydroxy-5-phenyl-3-pentyl)-2-(2-n-propoxyphenyl)-purin-6-on (Beispiel 17) hergestellt.
R_{f} = 0,49 (CH₂Cl₂ / CH₃OH 10:1)
Ausbeute: 28,5%

### Beispiel 28

9-(2-Methansulfonyloxy-3-nonyl)-2-(2-n-propoxy-5-morpholinosulfonyl-phenyl)-purin-6-on 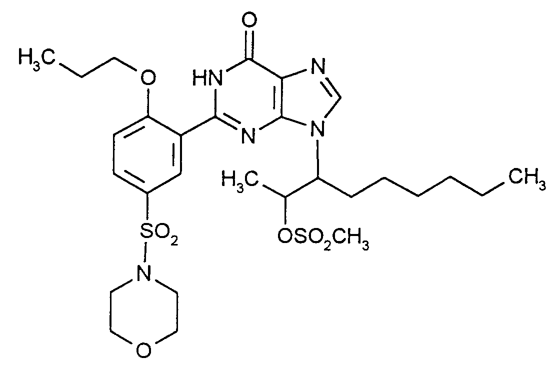
Die Titelverbindung wird in Analogie zur Vorschrift des Beispiels 22 ausgehend von 9-(2-Hydroxy-3-nonyl)-2-(2-n-propoxy-5-morpholinosulfonyl-phenyl)-purin-6-on (Beispiel 20) hergestellt.
R_{f} = 0,48 (CH₂Cl₂/CH₃OH 10:1)
Ausbeute: 51,4%

### Beispiel 29

9-(2-Azido-3-nonyl)-2-(2-n-propoxyphenyl)-purin-6-on 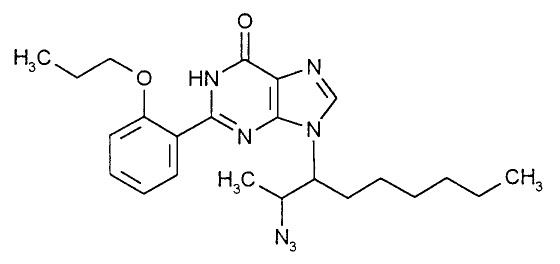
474 mg (1 mmol) 9-(2-Methansulfonyloxy-3-nonyl)-2-(2-n-propoxyphenyl)-purin-6-on (Beispiel 22) und 78 mg (1,2 mmol) Natriumazid werden in 5ml DMF 6 h (DC-Kontrolle) bei 100°C gerührt. Nach dem Abkühlen werden 20 ml Essigsäureethylester zugesetzt und 3 mal mit je 50 ml Wasser, 1 mal mit 50 ml gesättiger NaCl-Lösung ausgeschüttelt. Nach Trocknen über Na₂SO₄ wird die organische Phase im Vakuum eingedampft, und der Rückstand durch Flash-Chromatographie (Eluens: Toluol / Aceton 4:1) gereinigt.
R_{f} = 0,64 (CH₂Cl₂ / CH₃OH 10:1)
Ausbeute: 369 mg (88,4%)

### Beispiel 30

9-(2-Azido-5-phenyl-3-pentyl)-2-(2-n-propoxy-phenyl)-purin-6-on 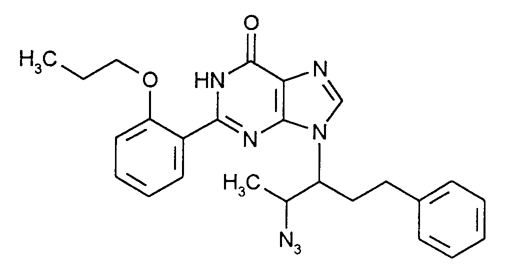
Die Titelverbindung wird in Analogie zur Vorschrift des Beispiels 29 ausgehend von 9-(2-Methansulfonyloxy-5-phenyl-3-pentyl)-2-(2-n-propoxyphenyl)-purin-6-on (Beispiel 23) hergestellt.
R_{f} = 0,54 (CH₂Cl₂ / CH₃OH 10:1)
Ausbeute: 87,4%

### Beispiel 31

9-(2-Azido-6-phenyl-3-hexyl)-2-(2-n-propoxyphenyl)-purin-6-on 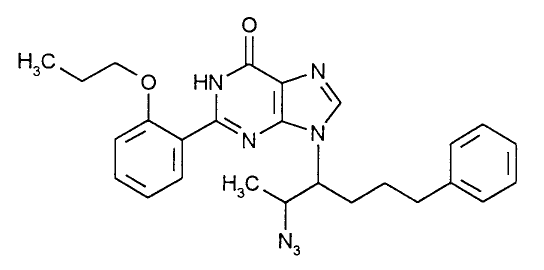
Die Titelverbindung wird in Analogie zur Vorschrift des Beispiels 29 ausgehend von 9-(2-Methansulfonyloxy-6-phenyl-3-hexyl)-2-(2-n-propoxyphenyl)-purin-6-on (Beispiel 24) hergestellt.
R_{f} = 0,55 (CH₂Cl₂ / CH₃OH 10:1)
Ausbeute: 74,9%

### Beispiel 32

9-(2-Azido-7-phenyl-3-heptyl)-2-(2-n-propoxyphenyl)-purin-6-on 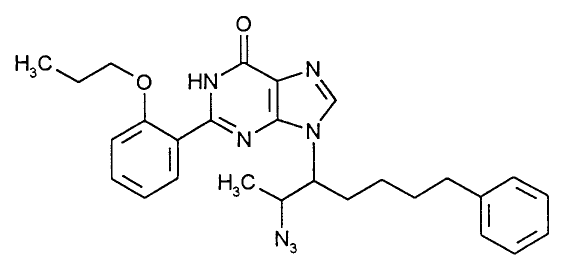
Die Titelverbindung wird in Analogie zur Vorschrift des Beispiels 29 ausgehend von 9-(2-Methansulfonyloxy-7-phenyl-3-heptyl)-2-(2-n-propoxyphenyl)-purin-6-on (Beispiel 25) hergestellt.
R_{f} = 0,6 (CH₂Cl₂ / CH₃OH 10:1)
Ausbeute: 67%

### Beispiel 33

9-(2-Azido-8-phenyl-3-octyl)-2-(2-n-propoxyphenyl)-purin-6-on 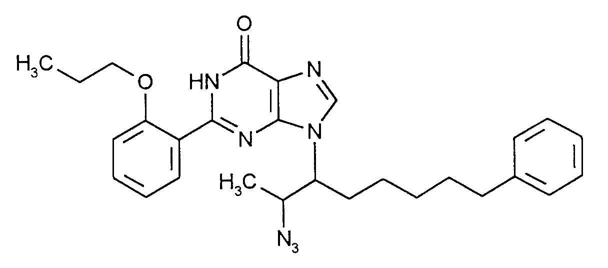
Die Titelverbindung wird in Analogie zur Vorschrift des Beispiels 29 ausgehend von 9-(2-Methansulfonyloxy-8-phenyl-3-octyl)-2-(2-n-propoxyphenyl)-purin-6-on (Beispiel 26) hergestellt.
R_{f} = 0,61 (CH₂Cl₂ / CH₃OH 10:1)
Ausbeute: 88%

### Beispiel 34

9-(1-Azido-5-phenyl-2-pentyl)-2-(2-n-propoxyphenyl)-purin-6-on 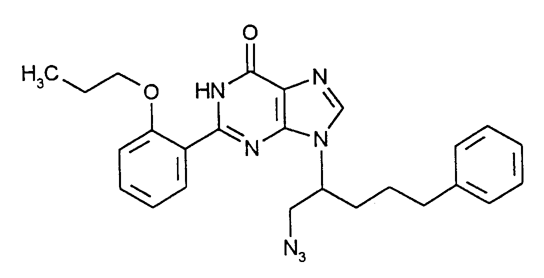
Die Titelverbindung wird in Analogie zur Vorschrift des Beispiels 29 ausgehend von 9-(1-Methansulfonyloxy-5-phenyl-2-pentyl)-2-(2-n-propoxyphenyl)-purin-6-on (Beispiel 27) hergestellt.
R_{f} = 0,56 (CH₂Cl₂ / MeOH 10:1)
Ausbeute: 98%

### Beispiel 35

9-(2-Azido-3-nonyl)-2-(2-n-propoxy-5-morpholinsulfonyl-phenyl)-purin-6-on 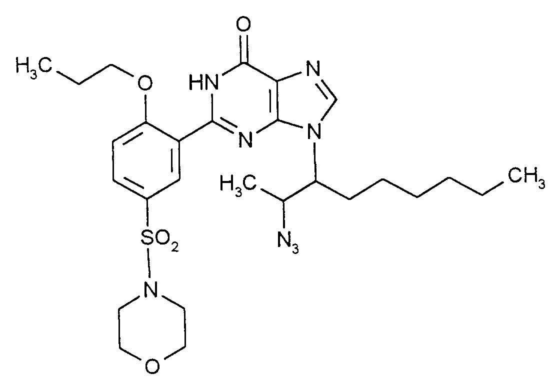
Die Titelverbindung wird in Analogie zur Vorschrift des Beispiels 29 ausgehend von 9-(2-Methansulfonyloxy-3-nonyl)-2-(2-n-propoxy-5-morpholinsulfonyl-phenyl)-purin-6-on (Beispiel 28) hergestellt.
R_{f} = 0,55 (CH₂Cl₂ / CH₃OH 10:1)
Ausbeute: 38%

### Beispiel 36

9-(2-Amino-3-nonyl)-2-(2-n-propoxyphenyl)-purin-6-on 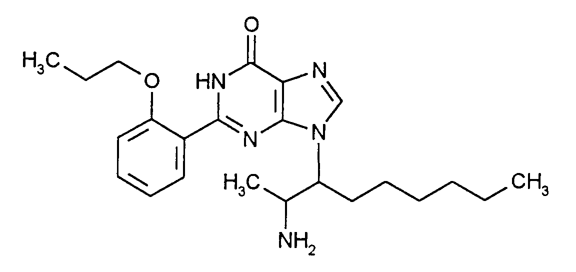
5,3 g (12,13 mmol) 9-(2-Azido-3-nonyl)-2-(2-n-propoxyphenyl)-purin-6-on (Beispiel 29) werden in 70 ml abs. Methanol in Gegenwart von 0,1 g Pd/C (10%) mit Wasserstoff bei Normaldruck und Raumtemperatur hydriert. Nach 4 h wird der Katalysator abfiltriert, das Lösemittel im Vakuum abdestilliert und der Rückstand über Kieselgel durch Flash-Chromatographie gereinigt (Eluens: CH₂Cl₂ / CH₃OH 20:1).
R_{f} = 0,28 (CH₂Cl₂ / CH₃OH 10:1)
Ausbeute: 4,1 g (82,3%)

### Beispiel 37

9-(2-Acetamino-3-nonyl)-2-(2-n-propoxyphenyl)-purin-6-on 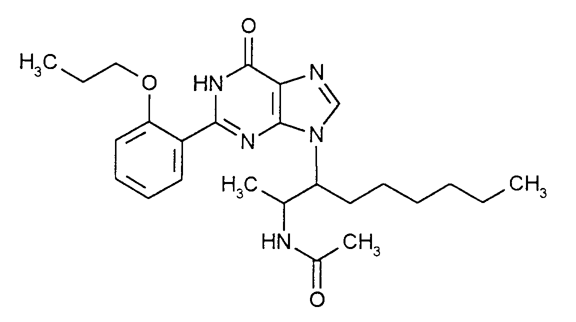
210 mg (0,5 mmol) 9-(2-Amino-3-nonyl)-2(2-n-propoxyphenyl)-purin-6-on (Beispiel 36) werden in 20 ml abs. CH₂Cl₂ gelöst. Es werden bei Raumtemperatur 101 mg (1 mmol) Triethylamin zugegeben; danach werden 78 mg (1 mmol) Acetylchlorid in 2 ml abs. CH₂Cl₂ zugetropft. Nach 1 h bei Raumtemperatur wird die organische Phase mit 10 ml 2 n HCl und mit 10 ml gesättigter NaHCO₃-Lösung ausgeschüttelt. Nach Trocknen der organischen Phase über Na₂SO₄ wird das Lösemittel im Vakuum eingedampft und der Rückstand durch Flashchromatographie gereinigt (Eluens: CH₂Cl₂ / CH₃OH 40:1).
R_{f} = 0,37 (CH₂Cl₂ / CH₃OH 10:1)
Ausbeute: 174 mg (77%)

### Beispiel 38

9-(2-Benzoylamino-3-nonyl)-2-(n-propoxyphenyl)-purin-6-on 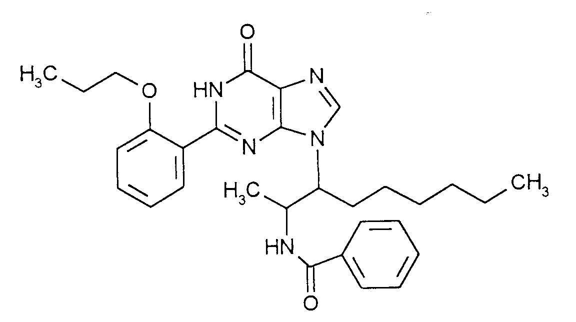
Die Titelverbindung wird in Analogie zur Vorschrift des Beispiels 37 ausgehend von 9-(2-Amino-3-nonyl)-2-(2-n-propoxyphenyl)-purin-6-on (Beispiel 36) und Benzoylchlorid hergestellt.
Fp.: 184°C (Toluol)
Ausbeute: 59%

### Beispiel 39

9-(2-Methylsulfonylamino-3-nonyl)-2-(2-n-propoxyphenyl)-purin-6-on 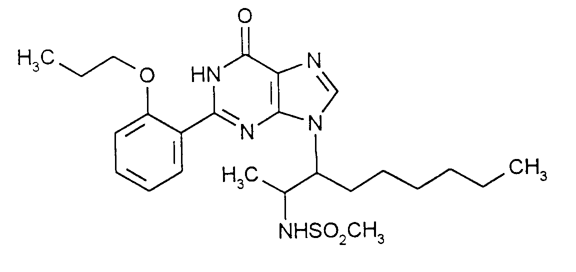
Die Titelverbindung wird in Analogie zur Vorschrift des Beispiels 37 ausgehend von 9-(2-Amino-3-nonyl)-2-(2-n-propoxyphenyl)-purin-6-on (Beispiel 36) und Methylsulfonylchlorid hergestellt.
R_{f}= 0,46 (CH₂Cl₂ / CH₃OH 10:1)
Ausbeute: 78,2%

### Beispiel 40

9-(2-Phenylsulfonylamino-3-nonyl)-2-(2-n-propoxyphenyl)-purin-6-on 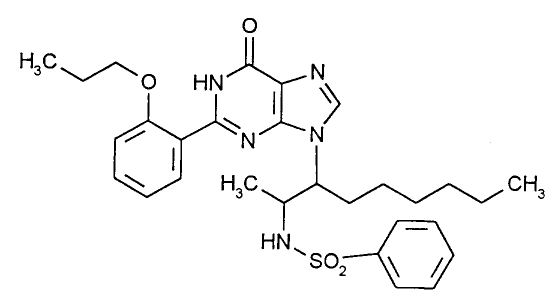
Die Titelverbindung wird in Analogie zur Vorschrift des Beispiels 37 ausgehend von 9-(2-Amino-3-nonyl)-2-(2-n-propoxyphenyl)-purin-6-on (Beispiel 36) und Phenylsulfonylchlorid hergestellt.
Fp.: 112°C (Toluol/Ether)
Ausbeute: 62,9%

### Beispiel 41

9-(2-Acetoxy-3-nonyl)-2-(2-n-propoxyphenyl)-purin-6-on 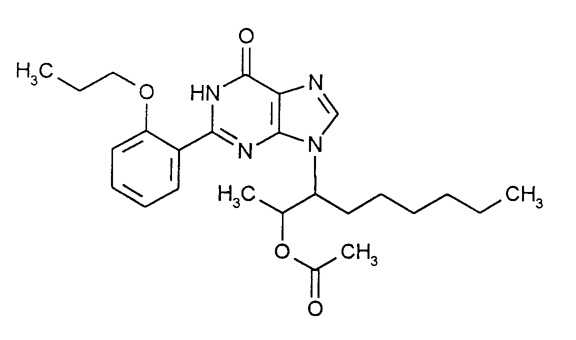
206 mg (0,5 mmol) 9-(2-Hydroxy-3-nonyl)-2-(2-n-propoxyphenyl)-purin-6-on (Beispiel 3), 47 mg (0,6 mmol) Acetylchlorid, 47 mg (0,6 mmol) Pyridin und 5 mg DMAP werden in 10 ml abs. CH₂Cl₂ 1 h bei 25°C, dann 1 h bei 40°C gerührt. Es wird 2 mal mit je 10 ml 2 n HCl und 2 mal mit je 10 ml gesättigter NaHCO₃-Lösung ausgeschüttelt und 1 mal mit gesättigter NaCl-Lösung gewaschen. Nach Trocknen der organischen Phase über Na₂SO₄ wird das Lösemittel im Vakuum eingedampft und der Rückstand im Hochvakuum getrocknet.
R_{f} = 0,53 (CH₂Cl₂ / CH₃OH 100:1)
Ausbeute: 90,5%

### Beispiel 42

9-(1-Acetoxy-2-octyl)-2-(2-n-propoxyphenyl)-purin-6-on 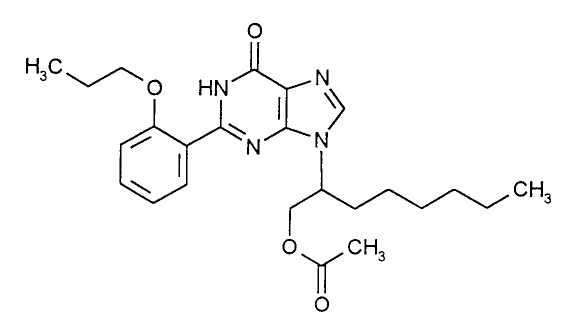
Die Titelverbindung wirdin Analogie zur Vorschrift des Beispiels 41 ausgehend von 9-(1-Hydroxy-2-octyl)-2-(2-n-propoxyphenyl)-purin-6-on (Beispiel 2) hergestellt.
R_{f} = 0,52 (CH₂Cl₂ / CH₃OH 100:1)
Ausbeute: 88,5%

### Beispiel 43

9-(2-Benzoyloxy-3-nonyl)-2-(2-n-propoxyphenyl)-purin-6-on 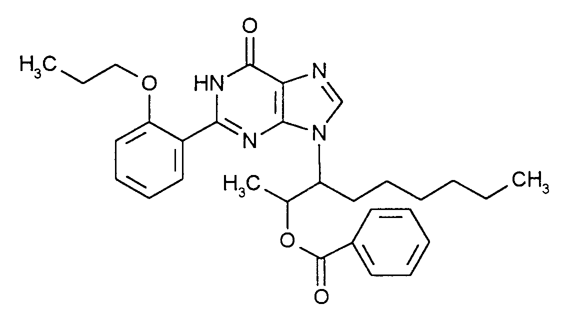
Die Titelverbindung wird in Analogie zur Vorschrift des Beispiels 41 ausgehend von 9-(2-Hydroxy-3-nonyl)-2-(2-n-propoxyphenyl)-purin-6-on (Beispiel 3) und Benzoylchlorid hergestellt.
R_{f} = 0,57 (CH₂Cl₂ / CH₃OH 100:1)
Ausbeute: 89%

### Beispiel 44

9-(2-Methoxy-3-nonyl)-2-(2-n-propoxyphenyl)-purin-6-on 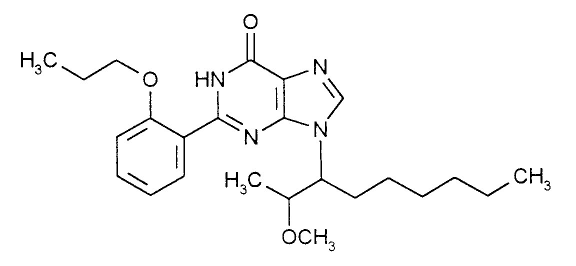
48 mg 60%iges NaH (1,2 mmol) werden bei 20°C in 2 ml abs. THF suspendiert. 206 mg (0,5 mmol) 9-(2-Hydroxy-3-nonyl)-2-(2-n-propoxyphenyl)-purin-6-on (Beispiel 3) in 3 ml abs. THF werden zugetropft. Nach 15 min bei 20°C werden 85 mg (0,6 mmol) Methyljodid in 3 ml abs. THF zugetropft und über Nacht bei 20°C gerührt. Das Lösemittel wird im Vakuum abrotiert, der Rückstand in 10 ml Essigsäureethylester aufgenommen und mit 20ml Wasser gewaschen. Nach Trocknen der organischen Phase mit Na₂SO₄ wird einrotiert und das Produkt durch Säulenchromatographie gereinigt (Eluens: Toluol / Aceton 5:1)
R_{f} = 0,58 (CH₂Cl₂ / CH₃OH 100:1)
Ausbeute: 74,2%

### Beispiel 45

9-(2-Ethoxycarbonylmethylenoxy-3-nonyl)-2(2-n-propoxyphenyl)-purin-6-on 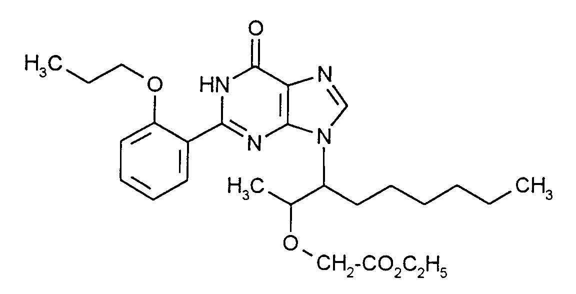
Die Titelverbindung wird in Analogie zur Vorschrift des Beispiels 44 ausgehend von 9-(2-Hydroxy-3-nonyl)-2-(2-n-propoxyphenyl)-purin-6-on (Beipsiel 3) und Bromessigsäureethylester hergestellt.
R_{f} = 0,55 (CH₂Cl₂ / CH₃OH 100:1)
Ausbeute: 85,8%

### Beispiel 46

9-(2-Carboxymethylenoxy-3-nonyl)-2-(2-n-propoxyphenyl)-purin-6-on 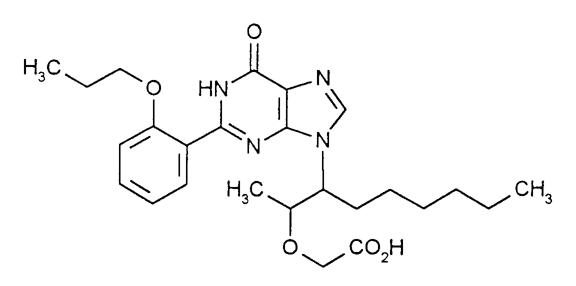
498 mg (1 mmol) des Esters aus Beispiel 45 werden 1 h bei 20°C in 4 ml 1 n NaOH und 5 ml MeOH gerührt Das Methanol wird im Vakuum abdestilliert. Nach Zugabe von 5 ml H₂O wird mit Essigsäureethylester ausgeschüttelt. Die wäßrige Phase wird mit 4 ml 2 n HCl angesäuert und 2 mal mit je 10 ml Essigsäureethylester ausgeschüttelt. Die vereinigten Essigsäureethylesterphasen werden über Na₂SO₄ getrocknet und eingedampft.
R_{f}= 0,27 (CH₂Cl₂ / CH₃OH 10:1)
Ausbeute: 88,5%

### Beispiel 47

9-(2-Amidocarbonylmethylenoxy-3-nonyl)-2-(2-n-propoxyphenyl)-purin-6-on 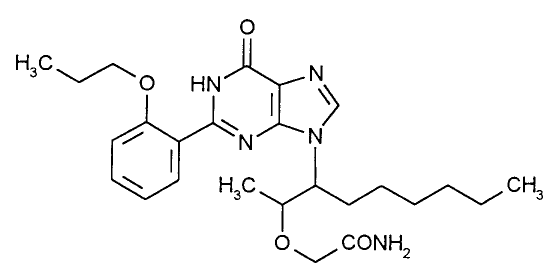
Die Titelverbindung wird in Analogie zur Vorschrift des Beispiels 45 ausgehend von 9-(2-Hydroxy-3-nonyl)-2-(2-n-propoxyphenyl)-purin-6-pn (Beispiel 3) und Bromessigsäureamid hergestellt.
R_{f} = 0,26 (CH₂Cl₂ / CH₃OH 10:1)
Ausbeute: 31,6%

### Beispiel 48

9-(2-Oxo-3-nonyl)-2-(2-n-propoxyphenyl)-purin-6-on 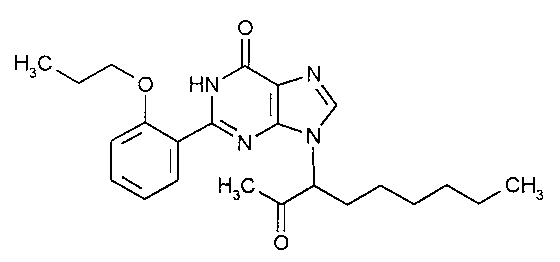
Zu 0,19 ml (2,2 mmol) Oxalylchlorid in 5 ml CH₂Cl₂ werden bei -60°C innerhalb von 10 min 0,31 ml abs. DMSO (4,4 mmol) in 3 ml abs. CH₂Cl₂ zugetropft und 20 min nachgerührt. Dann werden innerhalb von 45 min 824 mg (2 mmol) 9-(2-Hydroxy-3-methyl)-2-(2-n-propoxyphenyl)-purin-6-on (Beispiel 3) in 5 ml abs. CH₂Cl₂ zugetropft und 1 h bei -60°C nachgerührt. Zu dieser Lösung werden 1,39 ml (10 mmol) Triethylamin in 5 ml abs. CH₂Cl₂ innerhalb von 30 min zugetropft und 15 min bei -60°C nachgerührt. Man läßt auf 20°C kommen, gibt 10 ml H₂O zu, trennt die Phasen und wäscht die organische Phase mit 20 ml gesättigter NaCl-Lösung. Nach Trocknen über Na₂SO₄ wird eingedampft und der Rückstand durch Flashchromatographie (Eluens: CH₂Cl₂ / CH₃OH 40:1) gereinigt.
Fp.: 83°C (Ether / Cyclohexan)
Ausbeute: 535 mg (65,2%)

### Beispiel 49

9-(2-Ethyloximino-3-nonyl)-2-(2-n-propoxyphenyl)-purin-6-on 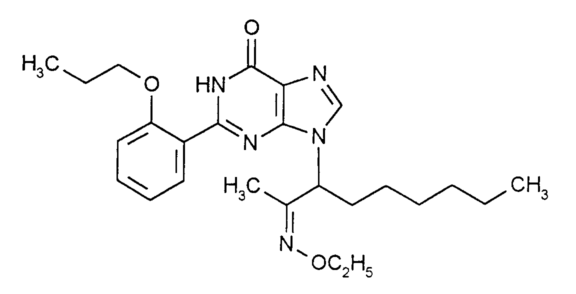
412 mg (1 mmol) 9-(2-Oxo-3-nonyl)-2-(2-n-propoxyphenyl)-purin-6-on (Beispiel 48) werden in 10 ml Methanol gelöst und mit 117 mg (1,2 mmol) Ethylhydroxylamin-hydrochlorid, gelöst in 1,5 ml Wasser, versetzt. Es wird 2 h am Rückfluß gekocht, abgekühlt und im Vakuum eingedampft. Der Rückstand wird in 10 ml Essigsäureethylester aufgenommen und mit 10 ml gesättigter NaHCO₃-Lösung gewaschen. Nach Trocknen der organischen Phase mit Na₂SO₄ wird das Lösemittel im Vakuum abdestilliert und der Rückstand durch Flashchromatographie (Eluens: Toluol / Aceton 4:1) gereinigt.
R_{f} = 0,53 (Toluol / Aceton 1:1)
Ausbeute: 366 mg (80,8%)
Nach dieser Vorschrift werden die in der Tabelle III aufgeführten Oxime durch Verwendung der entsprechenden Hydroxylamin-hydrochloride hergestellt (alle Oxime liegen als cis/trans-Gemische vor).

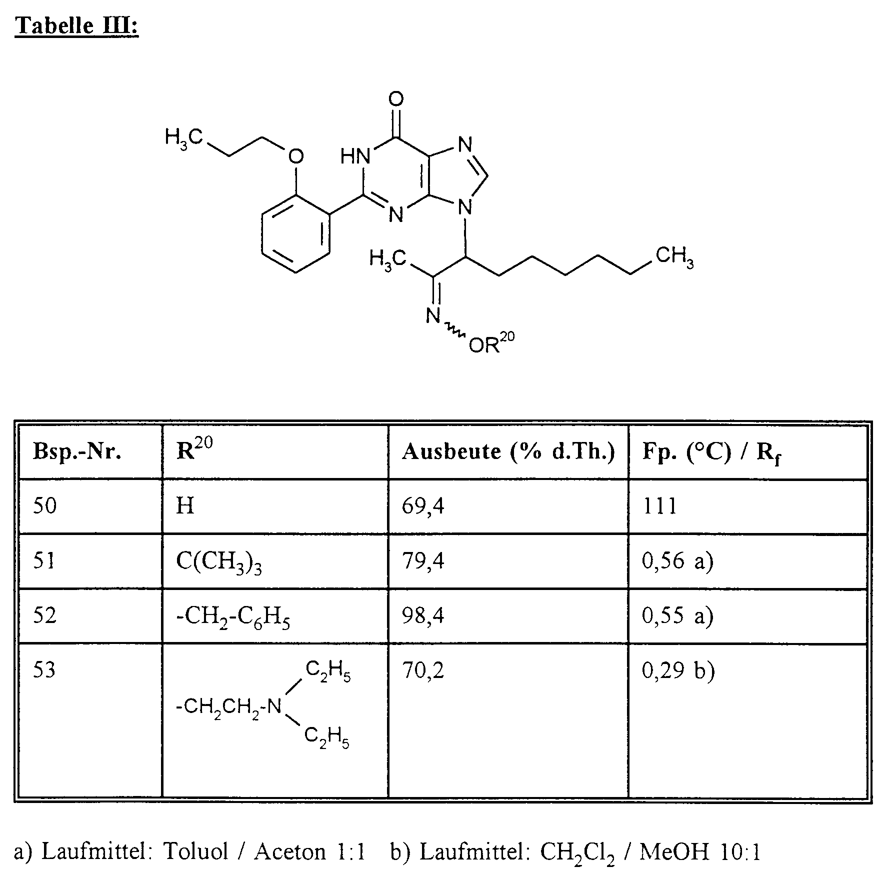

### Beispiel 54

9-(2-Acetoxy-3-nonyl)-2-(2-n-propoxy-5-diethylaminosulfonyl-phenyl)-purin-6-on 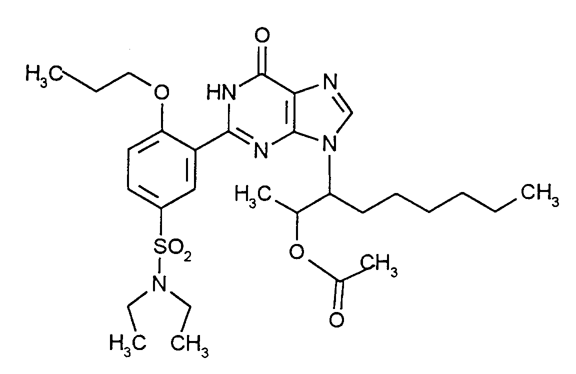
1,28 g (2,82 mmol) 9-(2-Acetoxy-3-nonyl)-2-(2-n-propoxyphenyl)-purin-6-on (Beispiel 41) werden portionsweise bei 0°C zu 4 ml Chlorsulfonsäure gegeben und über Nacht bei 20°C gerührt. Der Ansatz wird in 30 ml Eiswasser getropft und die wäßrige Phase wird 2 mal mit je 20 ml Essigsäureethylester extrahiert. Die vereinigten organischen Phasen werden über Na₂SO₄ getrocknet und das Lösemittel im Vakuum abdestilliert.
Ausbeute: 0,9 g (57,8%)
R_{f} = 0,52 (CH₂Cl₂ / CH₃OH 10:1)
Der Rückstand wird ohne weitere Reinigung in 30 ml abs. Ethanol aufgenommen, mit 5,1 ml Diethylamin versetzt und 6 h (DC-Kontrolle) bei 25°C gerührt. Das Ethanol wird im Vakuum abdestilliert, der Rückstand in 50 ml Essigsäureethylester aufgenommen und 2 mal mit je 50 ml H₂O gewaschen. Nach Trocknen über Na₂SO₄ wird die organische Phase im Vakuum eingedampft und der Rückstand durch Flashchromatographie (Eluens: Toluol/Aceton 3:1) gereinigt.
R_{f} = 0,52 (CH₂Cl₂ / CH₃OH 10:1)
Ausbeute: 549 mg (57,2%)
Die in der Tabelle IV aufgeführten Verbindungen werden in Analogie zur Vorschrift des Beispiels 54 aus dem entsprechenden Purinon und dem entsprechenden Amin hergestellt:

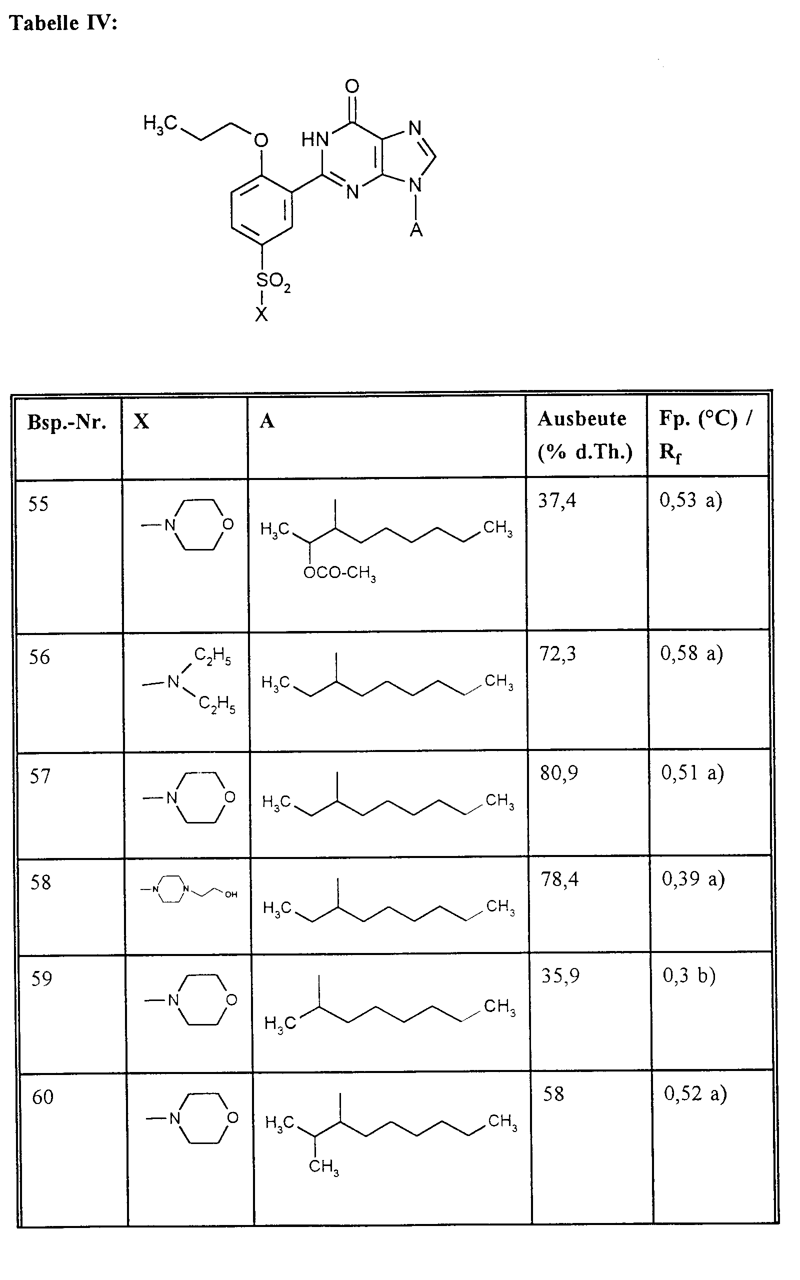

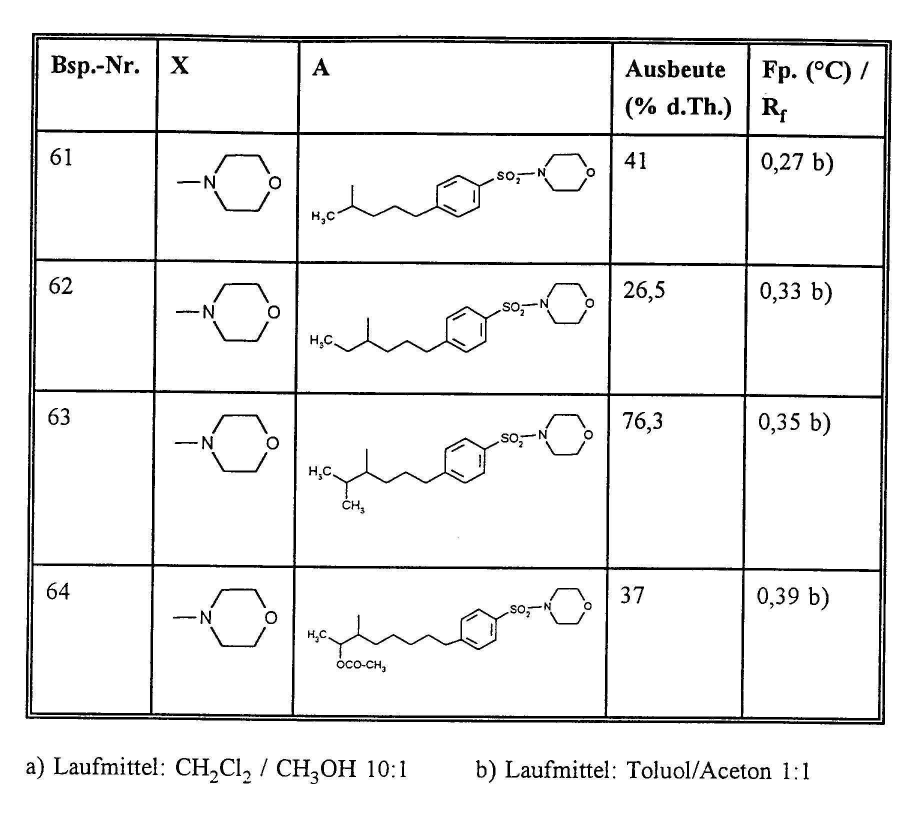

### Beispiel 65

9-(2-Hydroxy-3-nonyl)-2-(2-n-propoxy-5-diethylaminosulfonyl-phenyl)-purin-6-on 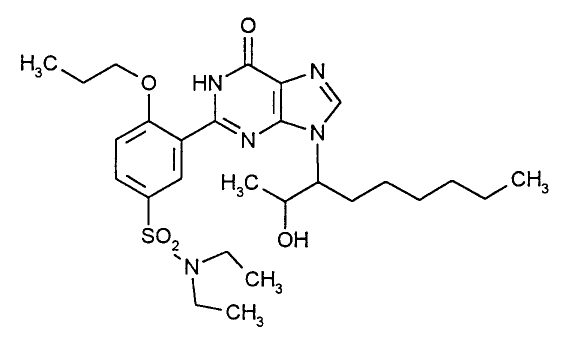
140 mg (0,24 mmol) 5-(2-Acetoxy-3-nonyl)-2-(2-n-propoxy-5-diethylaminosulfonyl-phenyl)-purin-6-on (Beispiel 54) werden in 5 ml Methanol gelöst. Nach Zugabe von 0,5 ml wäßriger 1 n NaOH Lösung wird 2 h bei 25°C gerührt. Es werden 0,25 ml wäßrige 2 n HCl Lösung zugeben, das Methanol im Vakuum abdestilliert und mit 10 ml Essigsäureethylester und 10 ml Wasser versetzt. Die organische Phase wird abgetrennt, über Na₂SO₄ getrocknet und eingedampft. Der Rückstand wird durch Flashchromatographie (Eluens: CH₂Cl₂ / CH₃OH 40:1) gereinigt.
R_{f} = 0,47 (CH₂Cl₂ / CH₃OH 10:1)
Ausbeute: 112 mg (85%)

### Beispiel 66

9-(2-Hydroxy-3-nonyl)-2-(2-n-propoxy-5-morpholinosulfonylphenyl)-purin-6-on 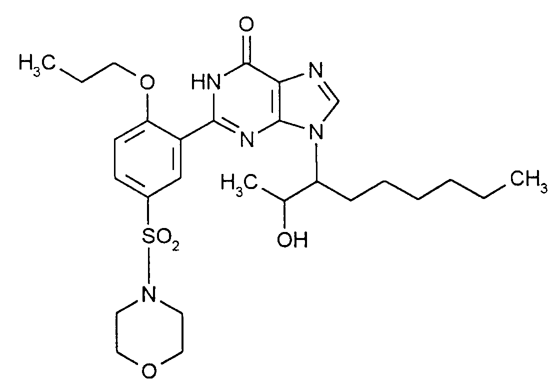
Die Titelverbindung wird in Analogie zur Vorschrift des Beispiels 65 ausgehend von 9-(2-Acetoxy-3-nonyl)-2-(2-n-propoxy-5-morpholinosulfonyl-phenyl)-purin-6-on (Beispiel 55) hergestellt.
R_{f} = 0,45 (CH₂Cl₂ / CH₃OH 10:1)
Ausbeute: 80,6%

### Beispiel 67

9-(2-Hydroxy-8-(4-morpholinosulfonyl-phenyl)-3-octyl)-2-(2-n-propoxy-5-morpholinosulfonyl-phenyl)-purin-6-on 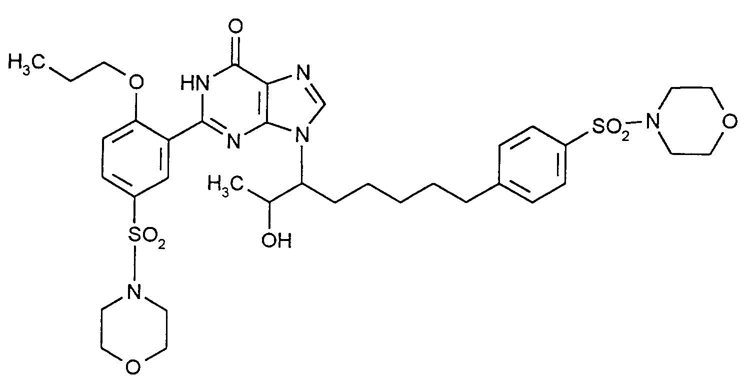
Die Titelverbindung wird in Analogie zur Vorschrift des Beispiels 65 ausgehend von 9-(2-Acetoxy-8-(4-morpholinosulfonyl-phenyl)-3-octyl)-2-(2-n-propoxy-5-morpholinosulfonyl-phenyl)-purin-6-on (Beispiel 64) hergestellt.
R_{f} = 0,35 (Toluol / Aceton 1:1)
Ausbeute: 79,6%

### Beispiel 68

9-(2-Oxo-3-nonyl)-2-(2-n-propoxy-5-morpholinosulfonyl-phenyl)-purin-6-on 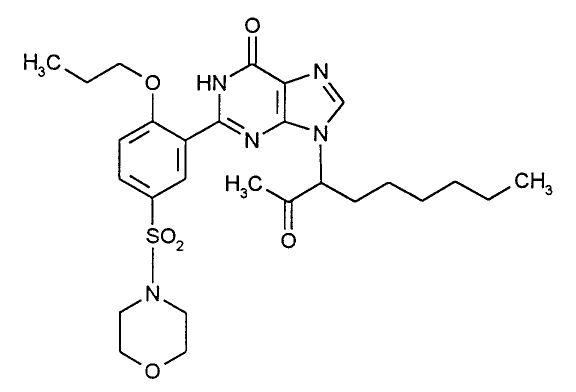
Die Titelverbindung wird in Analogie zur Vorschrift des Beispiels 48 ausgehend von 9-(2-Hydroxy-3-nonyl)-2-(2-n-propoxy-5-morpholinosulfonyl-phenyl)-purin-6-on (Beispiel 66) hergestellt.
R_{f} = 0,52 (CH₂Cl₂ / CH₃OH 10:19
Ausbeute: 63,2%

### Beispiel 69

9-(2-Oxo-8-(4-morpholinosulfonyl-phenyl)-3-octyl)-2-(2-n-propoxy-5-morpholinosulfonyl-phenyl)-purin-6-on 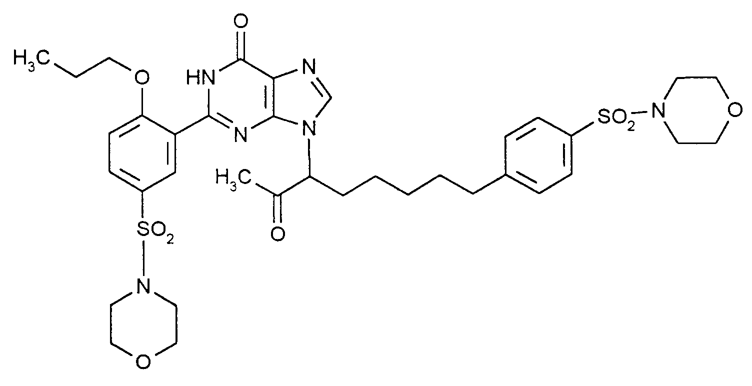
Die Titelverbindung wird in Analogie zur Vorschrift des Beispiels 48 ausgehend von 9-(2-Hydroxy-8-(4-morpholinosulfonyl-phenyl)-3-octyl)-2-(2-n-propoxy-5-morpholinosulfonyl-phenyl)-purin-6-on (Beispiel 67) hergestellt.
R_{f} = 0,41 (Toluol / Aceton 1:1)
Ausbeute: 51,1%

### Beispiel 70

9-(2-Oxo-6-phenyl-3-hexyl)-2-(2-n-propoxy-5-morpholinosulfonyl-phenyl)-purin-6-on 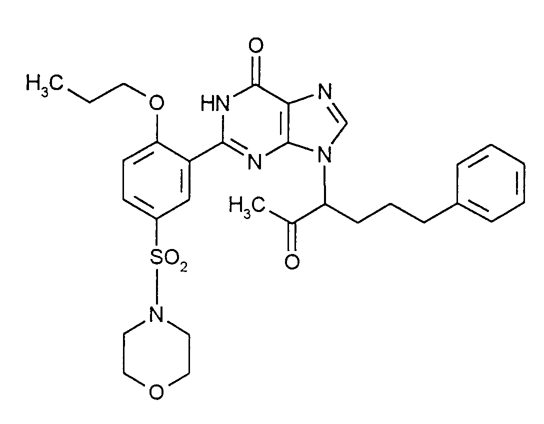
Die Titelverbindung wird in Analogie zur Vorschrift des Beispiels 48 ausgehend von 9-(2-Hydroxy-6-phenyl-3-hexyl)-2-(2-n-propoxy-5-morpholinosulfonyl-phenyl)-purin-6-on (Beispiel 20) hergestellt.
R_{f} = 0,6 (CH₂Cl₂ / CH₃OH 10:1)
Ausbeute: 51%

## Patentansprüche

1. 9-Substituierte 2-(2-n-Alkoxyphenyl)purin-6-one der allgemeinen Formel (I) 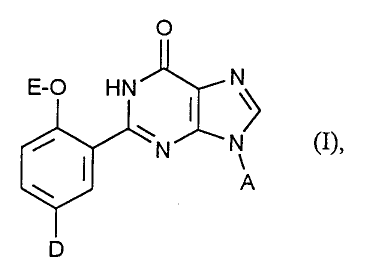 in welcher
A für einen Rest der Formel 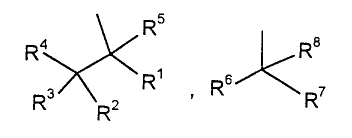 oder -(CH₂)ₐ-CH₃
steht,
worin
a eine Zahl 9, 10, 11, 12, 13, 14 oder 15 bedeutet,
R¹ geradkettiges oder verzweigtes Alkyl mit 2 bis 10 Kohlenstoffatomen bedeutet, das gegebenenfalls durch Phenyl substituiert ist, das seinerseits durch Halogen, Nitro, Cyano, durch geradkettiges oder verzweigtes Alkyl mit bis zu 6 Kohlenstoffatomen oder durch eine Gruppe der Formel -SO₂-NR⁹R¹⁰ substituiert sein kann,
worin
R⁹ und R¹⁰ gleich oder verschieden sind und Wasserstoff, Phenyl oder geradkettiges oder verzweigtes Alkyl mit bis zu 6 Kohlenstoffatomen bedeuten, das gegebenenfalls durch Hydroxy substituiert ist,
oder gemeinsam mit dem Stickstoffatom einen 5- bis 6-gliedrigen, gesättigten Heterocyclus mit bis zu 2 weiteren Heteroatomen aus der Reihe S, N und/oder O bilden, der gegebenenfalls, auch über eine freie N-Funktion, durch geradkettiges oder verzweigtes Alkyl mit bis zu 6 Kohlenstoffatomen substituiert ist, das seinerseits durch Hydroxy substituiert sein kann,
und/oder
Alkyl gegebenenfalls durch eine Gruppe der Formel -NR¹¹R¹² substituiert ist,
worin
R¹¹ und R¹² die oben angegebene Bedeutung von R⁹ und R¹⁰ haben und mit dieser gleich oder verschieden sind,
R² Wasserstoff, Azido, geradkettiges oder verzweigtes Alkyl mit bis zu 6 Kohlenstoffatomen oder eine Gruppe der Formel -OR¹³, O-SO₂R¹⁴ oder -NR¹⁵R¹⁶ bedeutet,
worin
R¹³ Wasserstoff, eine Hydroxyschutzgruppe, geradkettiges oder verzweigtes Acyl, mit bis zu 6 Kohlenstoffatomen, Benzoyl oder geradkettiges oder verzweigtes Alkyl mit bis zu 6 Kohlenstoffatomen bedeutet, das gegebenenfalls durch Carboxy oder geradkettiges oder verzweigtes Alkoxycarbonyl mit bis zu 6 Kohlenstoffatomen oder durch eine Gruppe der Formel -CO-NR¹⁷R¹⁸ substituiert ist,
worin
R¹⁷ und R¹⁸ gleich oder verschieden sind und Wasserstoff oder geradkettiges oder verzweigtes Alkyl mit bis zu 4 Kohlenstoffatomen bedeuten,
R¹⁴ geradkettiges oder verzweigtes Alkyl mit bis zu 4 Kohlenstoffatomen oder Phenyl bedeutet,
R¹⁵ und R¹⁶ gleich oder verschieden sind und Wasserstoff, eine Aminoschutzgruppe, geradkettiges oder verzweigtes Alkyl oder Acyl mit jeweils bis zu 6 Kohlenstoffatomen, Formyl, Benzoyl oder eine Gruppe der Formel -SO₂R¹⁹ bedeuten,
worin
R¹⁹ die oben angegebene Bedeutung von R¹⁴ hat und mit dieser gleich oder verschieden ist,
R³ Wasserstoff bedeutet,
oder
R² und R³ gemeinsam einen Rest der Formel =O oder -=N-OR²⁰ bilden,
worin
R²⁰ Wasserstoff oder geradkettiges oder verzweigtes Alkyl mit bis zu 6 Kohlenstoffatomen bedeutet, das gegebenenfalls durch Phenyl oder durch eine Gruppe der Formel -NR²¹R²² substituiert ist,
worin
R²¹ und R²² gleich oder verschieden sind und Wasserstoff, Phenyl oder geradkettiges oder verzweigtes Alkyl mit bis zu 6 Kohlenstoffatomen bedeuten,
R⁴ Wasserstoff oder geradkettiges oder verzweigtes Alkyl mit bis zu 4 Kohlenstoffatomen bedeutet,
R⁵ und R⁸ gleich oder verschieden sind und Wasserstoff oder geradkettiges oder verzweigtes Alkyl mit bis zu 3 Kohlenstoffatomen bedeuten,
R⁶ Wasserstoff oder geradkettiges oder verzweigtes Alkyl mit bis zu 5 Kohlenstoffatomen bedeutet, das gegebenenfalls durch Hydroxy substituiert ist,
R⁷ geradkettiges oder verzweigtes Alkyl mit 2 bis 8 Kohlenstoffatomen bedeutet, das durch eine Gruppe der Formel -NR²³R²⁴ substituiert ist,
worin
R²³ und R²⁴ gleich oder verschieden sind und Wasserstoff oder geradkettiges oder verzweigtes Alkyl mit bis zu 5 Kohlenstoffatomen bedeuten, das gegebenenfalls durch Hydroxy substituiert ist,
oder durch Phenyl substituiert ist, das seinerseits durch die Gruppe der Formel -SO₂-NR²⁵R²⁶ substituiert ist,
worin
R²⁵ und R²⁶ die oben angegebene Bedeutung von R⁹ und R¹⁰ haben,
D für Wasserstoff oder für eine Gruppe der Formel -SO₂NR²⁷R²⁸ steht,
worin
R²⁷ und R²⁸ gleich oder verschieden sind und die oben angegebene Bedeutung von R⁹ und R¹⁰ haben und mit dieser gleich oder verschieden sind,
E für geradkettiges oder verzweigtes Alkyl mit bis zu 8 Kohlenstoffatomen steht
und deren Tautomere und Salze.

2. 9-Substituierte 2-(2-n-Alkoxyphenyl)purin-6-one der Formel (I) nach Anspruch 1,
in welcher
A für einen Rest der Formel 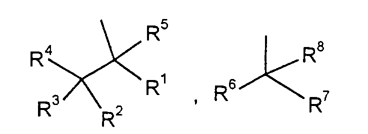 oder -(CH₂)ₐ-CH₃
steht,
worin
a eine Zahl 9, 10, 11, 12 oder 13 bedeutet,
R¹ geradkettiges oder verzweigtes Alkyl mit 2 bis 8 Kohlenstoffatomen bedeutet, das gegebenenfalls durch Phenyl substituiert ist, das seinerseits durch Fluor, Chlor, Brom, Nitro, Cyano, durch geradkettiges oder verzweigtes Alkyl mit bis zu 4 Kohlenstoffatomen oder durch eine Gruppe der Formel -SO₂-NR⁹R¹⁰ substituiert sein kann,
worin
R⁹ und R¹⁰ gleich oder verschieden sind und Wasserstoff, Phenyl oder geradkettiges oder verzweigtes Alkyl mit bis zu 5 Kohlenstoffatomen bedeuten, das gegebenenfalls durch Hydroxy substituiert ist,
oder gemeinsam mit dem Stickstoffatom einen Morpholinyl, Pyrrolidinyl-, Piperidinylring oder einen Piperazinylring bilden, der gegebenenfalls, auch über eine freie NH-Funktion, durch geradkettiges oder verzweigtes Alkyl mit bis zu 3 Kohlenstoffatomen substituiert ist, das seinerseits durch Hydroxy substituiert sein kann,
und/oder
Alkyl gegebenenfalls durch eine Gruppe der Formel -NR¹¹R¹² substituiert ist,
worin
R¹¹ und R¹² die oben angegebene Bedeutung von R⁹ und R¹⁰ haben und mit dieser gleich oder verschieden sind,
R² Wasserstoff, Azido, geradkettiges oder verzweigtes Alkyl mit bis zu 4 Kohlenstoffatomen oder eine Gruppe der Formel -OR¹³, -O-SO₂-R¹⁴ oder -NR¹⁵R¹⁶ bedeutet,
worin
R¹³ Wasserstoff, Benzyl, geradkettiges oder verzweigtes Acyl mit bis zu 4 Kohlenstoffatomen, Benzoyl oder geradkettiges oder verzweigtes Alkyl mit bis zu 4 Kohlenstoffatomen bedeutet, das gegebenenfalls durch Carboxy oder geradkettiges oder verzweigtes Alkoxycarbonyl mit bis zu 4 Kohlenstoffatomen oder durch eine Gruppe der Formel -CO-NR¹⁷R¹⁸ substituiert ist,
worin
R¹⁷ und R¹⁸ gleich oder verschieden sind und Wasserstoff, oder geradkettiges oder verzweigtes Alkyl mit bis zu 3 Kohlenstoffatomen bedeuten,
R¹⁴ geradkettiges oder verzweigtes Alkyl mit bis zu 3 Kohlenstoffatomen oder Phenyl bedeutet,
R¹⁵ und R¹⁶ gleich oder verschieden sind und Wasserstoff, tert. Butoxycarbonyl, Benzyloxycarbonyl oder geradkettiges oder verzweigtes Alkyl oder Acyl mit jeweils bis zu 4 Kohlenstoffatomen, Formyl, Benzoyl oder eine Gruppe der Formel -SO₂R¹⁹ bedeuten,
worin
R¹⁹ die oben angegebene Bedeutung von R¹⁴ hat und mit dieser gleich oder verschieden ist,
R³ Wasserstoff bedeutet,
oder
R² und R³ gemeinsam einen Rest der Formel =O oder -=N-OR²⁰ bilden,
worin
R²⁰ Wasserstoff oder geradkettiges oder verzweigtes Alkyl mit bis zu 4 Kohlenstoffatomen bedeutet, das gegebenenfalls durch Phenyl oder durch eine Gruppe der Formel -NR²¹R²² substituiert ist,
worin
R²¹ und R²² gleich oder verschieden sind und Wasserstoff, Phenyl oder geradkettiges oder verzweigtes Alkyl mit bis zu 4 Kohlenstoffatomen bedeuten,
R⁴ Wasserstoff oder geradkettiges oder verzweigtes Alkyl mit bis zu 3 Kohlenstoffatomen bedeutet,
R⁵ und R⁸ gleich oder verschieden sind und Wasserstoff oder Methyl bedeuten,
R⁶ Wasserstoff oder geradkettiges oder verzweigtes Alkyl mit bis zu 3 Kohlenstoffatomen bedeutet, das gegebenenfalls durch Hydroxy substituiert ist,
R⁷ geradkettiges oder verzweigtes Alkyl mit 2 bis 6 Kohlenstoffatomen bedeutet, das durch eine Gruppe der Formel -NR²³R²⁴ substituiert ist,
worin
R²³ und R²⁴ gleich oder verschieden sind und Wasserstoff oder geradkettiges oder verzweigtes Alkyl mit bis zu 4 Kohlenstoffatomen bedeuten, das gegebenenfalls durch Hydroxy substituiert ist,
oder durch Phenyl substituiert ist, das seinerseits durch eine Gruppe der Formel -SO₂-NR²⁵R²⁶ substituiert ist,
worin
R²⁵ und R²⁶ gleich oder verschieden sind und die oben angegebene Bedeutung von R⁹ und R¹⁰ haben,
D für Wasserstoff oder für eine Gruppe der Formel -SO₂-NR²⁷R²⁸ steht,
worin
R²⁷ und R²⁸ gleich oder verschieden sind und die oben angegebene Bedeutung von R⁹ und R¹⁰ haben und mit dieser gleich oder verschieden sind,
E für geradkettiges oder verzweigtes Alkyl mit bis zu 6 Kohlenstoffatomen steht,
und deren Tautomere und Salze.

3. 9-Substituierte 2-(2-n-Alkoxyphenyl)purin-6-one der Formel nach Anspruch 1,
worin
A für einen Rest der Formel 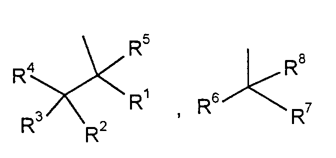 oder -(CH₂)ₐ-CH₃
steht,
worin
a eine Zahl 9, 10, 11 oder 12 bedeutet,
R¹ geradkettiges oder verzweigtes Alkyl mit 2 bis 7 Kohlenstoffatomen bedeutet, das gegebenenfalls durch Phenyl substituiert ist, das seinerseits durch Fluor, Chlor, Brom, Nitro, Cyano, durch geradkettiges oder verzweigtes Alkyl mit bis zu 3 Kohlenstoffatomen oder durch eine Gruppe der Formel -SO₂-NR⁹R¹⁰ substituiert sein kann,
worin
R⁹ und R¹⁰ gleich oder verschieden sind und Wasserstoff, Phenyl oder geradkettiges oder verzweigtes Alkyl mit bis zu 4 Kohlenstoffatomen bedeuten, das gegebenenfalls durch Hydroxy substituiert ist,
oder gemeinsam mit dem Stickstoffatom einen Morpholinyl, Pyrrolidinyl-, Piperidinylring oder einen Piperazinylring bilden, der gegebenenfalls, auch über eine freie NH-Funktion, durch geradkettiges oder verzweigtes Alkyl mit bis zu 3 Kohlenstoffatomen substituiert ist, das seinerseits durch Hydroxy substituiert sein kann,
und/oder
Alkyl gegebenenfalls durch eine Gruppe der Formel -NR¹¹R¹² substituiert ist,
worin
R¹¹ und R¹² die oben angegebene Bedeutung von R⁹ und R¹⁰ haben und mit dieser gleich oder verschieden sind,
R² Wasserstoff, Azido, geradkettiges oder verzweigtes Alkyl mit bis zu 3 Kohlenstoffatomen oder eine Gruppe der Formel -OR¹³, -OSO₂R¹⁴ oder -NR¹⁵R¹⁶ bedeutet,
worin
R¹³ Wasserstoff, geradkettiges oder verzweigtes Acyl mit bis zu 3 Kohlenstoffatomen, Benzoyl oder geradkettiges oder verzweigtes Alkyl mit bis zu 3 Kohlenstoffatomen bedeutet, das gegebenenfalls durch Carboxy oder geradkettiges oder verzweigtes Alkoxycarbonyl mit bis zu 3 Kohlenstoffatomen oder durch eine Gruppe der Formel -CO-NR¹⁷R¹⁸ substituiert ist,
worin
R¹⁷ und R¹⁸ gleich oder verschieden sind und Wasserstoff, Methyl oder Ethyl bedeuten,
R¹⁴ geradkettiges oder verzweigtes Alkyl mit bis zu 3 Kohlenstoffatomen oder Phenyl bedeutet,
R¹⁵ und R¹⁶ gleich oder verschieden sind und Wasserstoff, tert. Butoxycarbonyl, geradkettiges oder verzweigtes Alkyl oder Acyl mit jeweils bis zu 3 Kohlenstoffatomen, Formyl, Benzoyl oder eine Gruppe der Formel -SO₂R¹⁹ bedeuten,
worin
R¹⁹ die oben angegebene Bedeutung von R¹⁴ hat und mit dieser gleich oder verschieden ist,
R³ Wasserstoff bedeutet,
oder
R² und R³ gemeinsam einen Rest der Formel =O oder -=N-OR²⁰ bilden,
worin
R²⁰ Wasserstoff oder geradkettiges oder verzweigtes Alkyl mit bis zu 4 Kohlenstoffatomen bedeutet, das gegebenenfalls durch Phenyl oder durch eine Gruppe der Formel -NR²¹R²² substituiert ist,
worin
R²¹ und R²² gleich oder verschieden sind und Wasserstoff, Phenyl oder geradkettiges oder verzweigtes Alkyl mit bis zu 3 Kohlenstoffatomen bedeuten,
R⁴ Wasserstoff oder geradkettiges oder verzweigtes Alkyl mit bis zu 3 Kohlenstoffatomen bedeutet,
R⁵ und R⁸ gleich oder verschieden sind und Wasserstoff oder Methyl bedeuten,
R⁶ Wasserstoff oder geradkettiges oder verzweigtes Alkyl mit bis zu 3 Kohlenstoffatomen bedeutet, das gegebenenfalls durch Hydroxy substituiert ist,
R⁷ geradkettiges oder verzweigtes Alkyl mit 2 bis 6 Kohlenstoffatomen bedeutet, das durch eine Gruppe der Formel -NR²³R²⁴ substituiert ist,
worin
R²³ und R²⁴ gleich oder verschieden sind und Wasserstoff oder geradkettiges oder verzweigtes Alkyl mit bis zu 3 Kohlenstoffatomen bedeuten, das gegebenenfalls durch Hydroxy substituiert ist,
oder durch Phenyl substituiert ist, das seinerseits durch die Gruppe der Formel -SO₂-R²⁵R²⁶ substituiert ist,
worin
R²⁵ und R²⁶ die oben angegebene Bedeutung von R⁹ und R¹⁰ haben,
D für Wasserstoff oder für eine Gruppe der Formel -SO₂-NR²⁷R²⁸ steht,
worin
R²⁷ und R²⁸ gleich oder verschieden sind und die oben angegebene Bedeutung von R⁹ und R¹⁰ haben und mit dieser gleich oder verschieden sind,
E für geradkettiges oder verzweigtes Alkyl mit bis zu 5 Kohlenstoffatomen steht,
und deren Tautomere und Salze.

4. 9-Substituierte 2-(2-n-Alkoxyphenyl)purin-6-one nach Anspruch 1 bis 3, zur therapeutischen Anwendung.

5. Verfahren zur Herstellung von 9-substituierten 2-(2-n-Alkoxyphenyl)prin-6-onen nach Anspruch 1 bis 3, dadurch gekennzeichnet, daß man
zunächst Verbindungen der allgemeinen Formel (II) 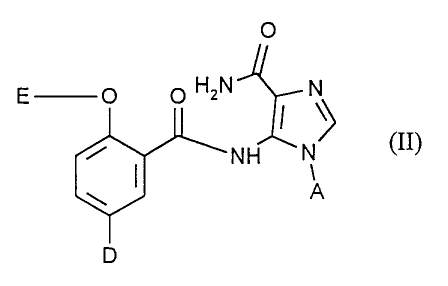 in welcher
D, E und A die oben angegebene Bedeutung haben,
in inerten Lösemitteln und in Anwesenheit einer Base cyclisiert,
und im Fall D = H zunächst durch Umsetzung mit Chlorsulfonsäure die entsprechenden Sulfonsäurechloride herstellt und anschließend mit Aminen (NR²⁵R²⁶) die entsprechenden Sulfonamide herstellt,
und die Substituenten R¹ - R⁸ nach üblichen Methoden, wie beispielsweise Alkylierung, Acylierung, Aminierung, Oxidation oder Azidaustausch einführt bzw. derivatisiert.

6. Arzneimittel enthaltend 9-substituierte 2-(2-n-Alkoxyphenyl)purin-6-one nach Anspruch 1 bis 3.

7. Arzneimittel nach Anspruch 6, zur Behandlung von Entzündungen, thromboembolischen Erkrankungen und Herz-/Kreislauferkrankungen.

8. Arzneimittel nach Anspruch 6 und 7 zur Behandlung von Impotenz.

9. Verwendung von 9-substituierte 2-(2-n-Alkoxyphenyl)purin-6-onen nach Anspruch 1 bis 3, zur Herstellung von Arzneimitteln.

10. Verwendung nach Anspruch 8, dadurch gekennzeichnet, daß es sich um Arzneimittel zur Behandlung von Entzündungen, thromboembolischen Erkrankungen und Herz-/Kreislauferkrankungen handelt.
